# FASCICULE DE BREVET EUROPEEN

(11) **EP 3 158 076 B1**
(45) Date de publication et mention de la délivrance du brevet: **16.06.2021**
(21) Numéro de dépôt: 15732869.1
(22) Date de dépôt: 12.06.2015
(51) Int. Cl.: C12P 19/14

(54) **VARIANTS D'EXOGLUCANASES A ACTIVITE AMELIOREE ET LEURS UTILISATIONS**
VARIANTEN VON EXOGLUCANASEN MIT VERBESSERTER AKTIVITÄT UND VERWENDUNGEN DAVON
VARIANTS OF EXOGLUCANASES HAVING IMPROVED ACTIVITY AND USES THEREOF

(30) Priorité: 20.06.2014 FR 1455701
(43) Date de publication de la demande: 26.04.2017
(73) Titulaire: IFP Energies nouvelles, 92500 Rueil-Malmaison (FR); Proteus, 91160 Longjumeau (FR); Centre National de la Recherche Scientifique (C.N.R.S.), 75016 Paris (FR)
(72) Inventeur: PERSILLON, Cécile, 30000 Nimes (FR); ULLMANN, Christophe, 30000 Nimes (FR); AYRINHAC, Céline, 30350 Domessargues (FR); BONZOM, Olivier, 30000 Nimes (FR); MARGEOT, Antoine, 75018 Paris (FR); MATHIS, Hugues, 77600 Bussy Saint Georges (FR); FORT, Sébastien, 38410 Vaulnaveys-le-Haut (FR); ARMAND, Sylvie, 38000 Grenoble (FR); PRADEAU, Stéphanie, 38350 Saint-Honoré (FR)
(74) Mandataire: Plasseraud IP
(86) Numéro de dépôt international: PCT/FR2015/051557
(87) Numéro de publication internationale: WO 2015/193588

(56) Documents cités:
- WO-A1-2014/081884
- WO-A1-2014/081884
- WO-A2-2014/078546
- FR-A1- 2 782 323
- DATABASE Geneseq [Online] 20 décembre 2012 (2012-12-20), "Nectria haematococca mpVI 77-13-4 protein, SEQ 13.", XP002732776, extrait de EBI accession no. GSP:BAG13688 Database accession no. BAG13688 -& WO 2012/149403 A1 (CODEXIS INC [US]; VOLADRI RAMA [US]; ZHANG XIYUN [US]; PATIL SACHIN [U) 1 novembre 2012 (2012-11-01)
- DATABASE Geneseq [Online] 22 janvier 2009 (2009-01-22), "Lignocellulosic enzyme sequence, SEQ ID 282.", XP002732777, extrait de EBI accession no. GSP:ASR93979 Database accession no. ASR93979
- DATABASE UniProt [Online] 13 June 2012 (2012-06-13), "RecName: Full=Glucanase {ECO:0000256|RuleBase:RU361186}; EC=3.2.1.- {ECO:0000256|RuleBase:RU361186};", retrieved from EBI accession no. UNIPROT:I1RIJ1 Database accession no. I1RIJ1

## Description

La possibilité de produire de l'éthanol à partir de la cellulose a reçu beaucoup d'attention en raison de la disponibilité de grandes quantités de matière première ainsi que de l'intérêt de l'éthanol à titre de carburant. Les matières premières naturelles cellulosiques pour un tel processus sont désignées par le terme "biomasse". De nombreux types de biomasse, par exemple le bois, les résidus agricoles, les cultures herbacées et les déchets solides municipaux, ont été considérés comme des matières premières potentielles pour la production de biocarburant. Ces matières sont constituées principalement de cellulose, d'hémicellulose et de lignine.

La cellulose est un polymère constitué de molécules de glucose reliées par des liens beta 1-4, qui sont très résistants à la dégradation ou à la dépolymérisation. Une fois la cellulose convertie en glucose, celui-ci est facilement fermenté en biocarburant, par exemple l'éthanol, en utilisant une levure.

Les plus anciennes méthodes étudiées pour convertir la cellulose en glucose sont basées sur l'hydrolyse acide. Ce processus peut se faire en présence d'acides concentrés ou dilués. Cependant, plusieurs inconvénients tels que la mauvaise récupération de l'acide lors de l'utilisation d'acides concentrés et la faible production de glucose dans le cadre de l'utilisation d'acides dilués nuisent à l'économie du processus d'hydrolyse acide.

Pour surmonter les inconvénients du processus d'hydrolyse acide, les processus de conversion de la cellulose ont porté plus récemment sur l'hydrolyse enzymatique, à l'aide d'enzymes de type cellulase. Cette hydrolyse enzymatique de la biomasse lignocellulosique (par exemple, la cellulose) présente cependant l'inconvénient d'être un procédé industriel coûteux. De ce fait, il est nécessaire d'utiliser des souches de microorganismes sécréteurs de cellulases de plus en plus performantes. À ce titre, beaucoup de microorganismes comportent des enzymes qui hydrolysent la cellulose, tels que les champignons *Trichoderma, Aspergillus, Humicola, Fusarium* ainsi que des bactéries telles que *Thermomonospora, Bacillus, Cellulomonas* et *Streptomyces.* Les enzymes sécrétées par ces microorganismes possèdent trois types d'activités utiles dans la conversion de la cellulose en glucose et se divisent en trois groupes: les endoglucanases, qui attaquent les fibres de celluloses aléatoirement en interne, les exoglucanases qui vont attaquer les extrémités des fibres en libérant du cellobiose, et les beta-glucosidases qui vont hydrolyser ce cellobiose en glucose. D'autres classes d'enzymes telles que les hémicellulases ou la classe d'enzymes récemment découverte des polysaccharides mono-oxygénases peuvent jouer également un rôle dans l'efficacité de l'hydrolyse.

Il y a un intérêt industriel fort pour la diminution du coût de l'hydrolyse enzymatique, et cette diminution passe par l'utilisation d'une dose réduite d'enzymes et donc des cocktails d'enzymes plus efficaces. En conséquence, plusieurs demandes de brevets décrivent des enzymes naturelles aux capacités supérieures à celles de *Trichoderma reesei,* ou des variants améliorés par génie génétique. On peut citer les demandes de brevet US2010304464, WO2010066411, WO2014078546, WO2012149403, et WO2013029176 concernant les exoglucanases, les demandes WO2007109441, WO2012149192 et WO2010076388 concernant les endoglucanases, les demandes WO2010029259, WO2010135836 ou WO2010022518 concernant les beta-glucosidases, ou encore les demandes WO12135659, WO12149344 concernant les polysaccharides mono-oxygénases. On peut également citer les numéro d'accession suivants concernant les exoglucanases : BBG53179, BAG13668. La référence ASR93979 décrit quant à elle une enzyme ayant une activité lignocellulosique.

Le numéro d'accession Uniprot I1RIJ1 fait référence à une protéine ayant une activité glucanase putative.

La demande de brevet FR2782323 décrit un procédé de production in vitro de séquences polynucléotidiques recombinées.

La demande WO2014081884 décrit des protéines sécrétées modifées.

Les enzymes hydrolysant la biomasse lignocellulosique sont classées dans le système CAZy (Cantarel, B. L., Coutinho, P. M., Rancurel, C., Bernard, T., Lombard, V., & Henrissat, B. (2009). The Carbohydrate-Active EnZymes database (CAZy): an expert resource for Glycogenomics. Nucleic acids research, 37, D233-8) sur des critères principalement structuraux. Les exoglucanases peuvent appartenir aux familles GH 6, 7, 9, 48, et 74.

Pour qu'une hydrolyse de la biomasse lignocellulosique soit efficace et économiquement rentable, le mélange enzymatique doit comporter des proportions équilibrées d'enzymes ayant des activités enzymatiques diverses, entre autres, mais non exclusivement, du type exoglucanases, endoglucanases, xylanases et beta-glucosidases. A titre d'exemple, dans les mélanges natifs de *Trichoderma reesei* on constate généralement la présence de 60-70% d'exoglucanases, 15-20% d'endoglucanases, quelques pourcentages d'hémicellulases et environ 5-10% de beta-glucosidases. Ce mélange convient pour hydrolyser la majorité des substrats prétraités (ex. type paille de blé explosée à la vapeur en conditions acides) avec des rendements acceptables. La proportion déjà importante des exoglucanases dans le mélange indique qu'il sera difficile d'augmenter la quantité de ces enzymes sans pénaliser les autres activités. Le génome de *Trichoderma reesei* comporte deux exoglucanases, l'une issue de la famille 6 (CBH2, cel6a) et l'autre issue de la famille 7 (CBH1, Cel7a). Elles hydrolysent en cellobiose respectivement les extrémités non réductrices (EC3.2.1.176) et réductrices (EC3.2.1.91) de la cellulose.

L'hydrolyse et la fermentation peuvent être réalisées suivant différents schémas. Le plus courant consiste en une hydrolyse et une fermentation séparées (SHF - Separate Hydrolysis and Fermentation). Cette méthode permet d'optimiser chaque étape par le maintien des conditions optimales de réaction. Cette fermentation s'effectue de manière extemporanée, à une température comprise entre environ 28°C et environ 30°C, tandis que l'hydrolyse a lieu généralement à une température d'au moins 45°C. Cependant, en SHF, les sucres libérés en fin de réaction sont présents à très forte concentration et entraînent une inhibition des enzymes, ralentissant l'efficacité du procédé. Pour éviter ces inconvénients, un autre type de procédé peut être envisagé. En SSF, les deux étapes (hydrolyse et fermentation des hexoses) ont lieu de manière simultanée, empêchant l'accumulation des sucres à des concentrations inhibitrices pour les enzymes. Les coûts d'investissement sont également réduits grâce à l'utilisation d'un seul réacteur. Le taux d'hydrolyse est plus élevé suite à l'absence d'inhibition car les sucres libérés sont utilisés immédiatement pour la fermentation en éthanol. Dans cette méthode, la température du réacteur constitue nécessairement un compromis entre les températures optimales d'hydrolyse et de fermentation, typiquement entre environ 30°C et environ 35°C. Cependant, à une telle température, l'activité des enzymes cellulolytiques est diminuée de 30% environ.

La SSF permet également l'expression d'enzymes dégradant la cellulose dans l'organisme fermentant les sucres, ce qui permet de limiter, ou dans un cas extrême de supprimer le recours aux enzymes produites lors d'une étape séparée.

En conséquence, l'obtention d'enzymes maintenant une activité exoglucanase efficace aux températures optimales d'hydrolyse et de fermentation (soit entre 30°C et 50°C) tout en gardant la proportion de l'ensemble des enzymes du mélange serait un gain significatif pour le procédé de conversion de biomasse lignocellulosique en biocarburant.

Les inventeurs ont développé un polypeptide ayant une activité exoglucanase améliorée, notamment par rapport à l'activité exoglucanase de la protéine de référence CBH2 de séquence SEQ ID NO:2. CBH2 correspond à l'exoglucanase 2 de *Trichoderma reesei.*

Dans cette optique, les déposants, ont eu le grand mérite de trouver, après de nombreuses recherches, un polypeptide isolé ou purifié ayant une activité exoglucanase améliorée par rapport à l'activité exoglucanase de la protéine de référence CBH2 (SEQ ID NO :2).

La description concerne donc un polypeptide choisi dans le groupe consistant en :
i. une séquence d'acides aminés choisie parmi SEQ ID NO :4, SEQ ID NO :6, SEQ ID NO :8, SEQ ID NO :10, SEQ ID NO :12, SEQ ID NO :14, SEQ ID NO :16, SEQ ID NO :18, SEQ ID NO :20, SEQ ID NO :22, SEQ ID NO :24, SEQ ID NO :26 et SEQ ID NO :28 ; et
ii. une séquence d'acides aminés présentant un pourcentage de résidus identiques par rapport à la séquence SEQ ID NO :6, SEQ ID NO :8, SEQ ID NO :10, SEQ ID NO :12, SEQ ID NO :14, SEQ ID NO :16, SEQ ID NO :18, SEQ ID NO :20, SEQ ID NO :22, SEQ ID NO :24, SEQ ID NO :26 et SEQ ID NO :28, (pourcentage d'identité) d'au moins 70%, préférentiellement d'au moins 75%, 80%, 85%, 90%, 95%, 98% ou 99%.

L'invention est définie par les revendications.

De préférence, le polypeptide tel que décrit précédemment est caractérisé en ce que son expression dans un organisme fermentaire est au moins égale à l'expression de la protéine de référence CBH2 (SEQ ID NO :2).

Selon l'invention, le pourcentage d'identité d'une séquence donnée par rapport à SEQ ID NO :4, 6, 8, 10, 12, 14, 16, 18, 20, 22, 24, 26 ou 28 correspond au nombre de résidus identiques entre cette séquence donnée et SEQ ID NO :4, 6, 8, 10, 12, 14, 16, 18, 20, 22, 24, 26 ou 28 divisé par le nombre de résidus dans SEQ ID NO :4, 6, 8, 10, 12, 14, 16, 18, 20, 22, 24, 26 ou 28.

eLe polypeptide de l'invention a une activité exoglucanase améliorée d'au moins 10%, préférentiellement d'au moins 20%, préférentiellement d'au moins 30%, encore plus préférentiellement d'au moins 40%, à une température d'environ 35°C et/ou d'environ 50°C, par rapport à l'activité exoglucanasse du polypeptide CBH2 de séquence d'acides aminés SEQ ID N°2.

L'homme du métier pourra par exemple déterminer l'augmentation ou autrement dit l'amélioration de l'activité enzymatique soit à l'aide d'un substrat comme la cellulose Avicel®, la cellulose PASC ou avec un substrat chromogénique (p-Nitrophenyl glycoside), par exemple le pNP lactose. L'activité enzymatique sera respectivement révélée par dosage colorimétrique des sucres réducteurs ou bien du nitrophénol libérés.

Un exemple de protocole, que l'homme du métier pourra utiliser pour déterminer si un polypeptide selon l'invention présente une activité enzymatique améliorée par rapport à celle de la protéine de référence CBH2 (SEQ ID NO :2), est le suivant :
- préparation d'une culture stock de *Y. lipolytica* exprimant une enzyme recombinante selon l'invention pendant toute la nuit à 28°C ;
- ensemencement d'un milieu d'expression avec un volume de culture stock permettant d'avoir une densité optique à 600 nm égale à 0.2 au début de la culture ;
- culture desdites cellules à 28°C pendant 96 heures ;
- centrifugation à 8000 rpm pendant 5 minutes ;
- incubation de 100 µL de surnageant avec 100 µL de tampon citrate phosphate 0.1 M pH 6 contenant 1 % de cellodextrines (CD) réduites pendant 4 heures à 35°C et 50°C ;
- prélèvement de 100 µL de réaction ;
- ajout de 100 µL de réactif DNS ;
- incubation 5 minutes à 100°C ;
- incubation 3 minutes sur la glace ;
- centrifugation 10 minutes à 3000 rpm ;
- lecture de la DO à 540 nm sur 150 µL.

L'invention a également pour objet, un acide nucléique purifié ou isolé codant au moins un polypeptide tel que décrit précédemment, selon l'invention. Le TABLEAU 1 ci-dessous comprend les identifications des séquences nucléiques et peptidiques pour le gène de référenceCBH2 de *T. reesei* , les exoglucanases putatives de *Nectria haematococca* (NH) et de *Giberella zeae* (GZ), ainsi que pour les polypeptides et polynucléotides de l'invention.

**TABLEAU 1**

| **Clones** | **Acide nucléique** | **Polypeptide** |
|---|---|---|
| CBH2 (sauvage) | SEQ ID NO :1 | SEQ ID NO :2 |
| 35B7 | SEQ ID NO :3 | SEQ ID NO :4 |
| 95B7 | SEQ ID NO :5 | SEQ ID NO :6 |
| 100F11 | SEQ ID NO :7 | SEQ ID NO :8 |
| 139F12 | SEQ ID NO :9 | SEQ ID NO :10 |
| 157B11 | SEQ ID NO :11 | SEQ ID NO :12 |
| 161A1 | SEQ ID NO :13 | SED ID NO :14 |
| 161C12 | SEQ ID NO :15 | SED ID NO :16 |
| 189H8 | SEQ ID NO :17 | SEQ ID NO :18 |
| 196D9 | SEQ ID NO :19 | SEQ ID NO :20 |
| 198E11 | SEQ ID NO :21 | SEQ ID NO :22 |
| 251B4 | SEQ ID NO :23 | SEQ ID NO :24 |
| 251C4 | SEQ ID NO :25 | SEQ ID NO :26 |
| 382A2 | SEQ ID NO :27 | SED ID NO :28 |
| Gène GZ | SEQ ID NO :29 | SEQ ID NO :30 |
| Gène NH-7 | SEQ ID NO :31 | SED ID NO :32 |

L'invention a également pour objet un acide nucléique purifié ou isolé codant au moins un polypeptide tel que décrit précédemment, selon l'invention.

De préférence, ledit acide nucléique purifié ou isolé peut être choisi parmi les séquences suivantes: SEQ ID NO :3, SEQ ID NO :5, SEQ ID NO :7, SEQ ID NO :9, SEQ ID NO :11; SEQ ID NO :13, SEQ ID NO :15, SEQ ID NO :17, SEQ ID NO :19, SEQ ID NO :21, SEQ ID NO :23, SEQ ID NO :25 et SEQ ID NO :27.

Selon l'invention, l'acide nucléique tel que décrit précédemment pourra être lié opérationnellement à un promoteur, un terminateur ou toute autre séquence nécessaire à son expression dans une cellule hôte.

L'invention porte également sur un vecteur comprenant au moins un acide nucléique tel que décrit précédemment, selon l'invention.

Selon l'invention, on entend par « vecteur » toute séquence d'ADN dans laquelle il est possible d'insérer des fragments d'acide nucléique étranger, les vecteurs permettant d'introduire de l'ADN étranger dans une cellule hôte. On peut citer de manière non-exhaustive comme vecteurs : les plasmides, les cosmides, les chromosomes artificiels de levures (YAC), les chromosomes artificiels de bactéries (BAC), les chromosomes artificiels dérivés du bactériophage P1 (PAC) ou les vecteurs dérivés de virus.

Le vecteur selon l'invention pourra également porter un marqueur de sélection. On entend par « marqueur de sélection » un gène dont l'expression confère aux cellules qui le contiennent une caractéristique permettant de les sélectionner. Il s'agit par exemple d'un gène de résistance aux antibiotiques.

L'invention a également pour objet une cellule hôte isolée comprenant soit au moins l'un des polypeptides tels que décrit précédemment, selon l'invention, soit au moins l'un des acides nucléiques tels que décrit précédemment, selon l'invention, soit au moins l'un des vecteurs tels que décrits précédemment, selon l'invention.

L'homme du métier pourra introduire l'un des polypeptides, l'un des acides nucléiques ou l'un des vecteurs tels que décrits précédemment dans la cellule hôte par des méthodes conventionnelles bien connues. Par exemple, on peut citer le traitement au chlorure de calcium, l'électroporation, l'utilisation d'un pistolet à particules.

Selon un mode de réalisation, l'homme du métier pourra introduire dans la cellule hôte et par des méthodes conventionnelles plusieurs copies d'un acide nucléique codant un polypeptide ayant une activité exoglucanase améliorée selon l'invention.

Selon un mode de réalisation, la cellule hôte isolée telle que décrite précédemment selon l'invention est choisie parmi *Trichoderma, Aspergillus, Neurospora, Humicola, Myceliophthora, Chrysosporium, Penicillium, Fusarium, Thermomonospora, Bacillus, Pseudomonas, Escherichia, Clostridium, Cellulomonas, Streptomyces, Yarrowia, Pichia* et *Saccharomyces.*

Selon un mode de réalisation préféré, la cellule hôte isolée telle que décrite précédemment selon l'invention est choisie parmi *Trichoderma reesei, Trichoderma viridae, Trichoderma koningii, Aspergillus niger, Aspergillus nidulans, Aspergillus wentii, Aspergillus oryzae, Aspergillus phoenicis, Myceliophthora thermopila, Chrysosporium lucknowense, Neurospora crassa, Humicola grisae, Penicillium pinophilum, Penicillium oxalicum, Escherichia coli, Clostridium acetobutylicum, Clostridium saccharolyticum, Clostridium benjerinckii, Clostridium butylicum, Pichia pastoris, Yarrowia lipolityca* et *Saccharomyces cerevisiae.*

Selon un mode de réalisation préféré, la cellule hôte isolée telle que décrite précédemment selon l'invention est choisie parmi *Trichoderma reesei* et *Saccharomyces cerevisiae.*

L'invention a également pour objet l'utilisation de l'un quelconque des polypeptides décrits précédemment, selon l'invention, pour l'hydrolyse de la cellulose.

L'invention a également pour objet, l'utilisation de l'un quelconque des polypeptides décrits précédemment, selon l'invention, pour la production de biocarburant.

Selon l'invention, le terme biocarburant peut être défini comme étant tout produit issu de la transformation de la biomasse et pouvant être utilisé à des fins énergétiques. D'une part et sans vouloir se limiter, on peut citer à titre d'exemple des biogaz, des produits pouvant être incorporés (éventuellement après transformation ultérieure) à un carburant ou être un carburant à part entière, tels que des alcools (l'éthanol, le butanol et/ou l'isopropanol selon le type d'organisme fermentaire utilisé), des solvants (acétone), des acides (butyrique), des lipides et leurs dérivés (acides gras à courtes ou longues chaînes, esters d'acides gras), ainsi que l'hydrogène.

De manière préférée, le biocarburant selon l'invention est un alcool, par exemple l'éthanol, le butanol et/ou l'isopropanol. Plus préférentiellement, le biocarburant selon l'invention est l'éthanol.

Dans un autre mode de réalisation, le biocarburant est du biogaz.

Dans un autre mode de réalisation, le produit est une molécule intéressant l'industrie chimique, comme par exemple, un autre alcool tel que le 1,2-propane diol, le 1,3-propane diol, le 1,4-butane diol, le 2,3-butane diol, des acides organiques comme l'acide acétique, propionique, acrylique, butyrique, succinique, malique, fumarique, citrique, itaconique, ou des hydroxyacides comme l'acide glycolique, hydroxypropionique, ou lactique.

On décrit ci-dessous un mode de réalisation de production d'un cocktail enzymatique utile pour l'hydrolyse de la lignocellulose.

Les souches de champignons filamenteux, de préférence *Trichoderma,* plus préférentiellement *T. reesei,* capables d'exprimer au moins un polypeptide selon l'invention sont cultivées en fermenteurs, en présence d'un substrat carboné, tel que le lactose ou le glucose, choisi pour la croissance du microorganisme. Dans un mode de réalisation, ce substrat carboné, selon sa nature, est introduit dans le fermenteur avant stérilisation ou est stérilisé séparément et introduit dans le fermenteur après stérilisation de ce dernier pour obtenir une concentration initiale de 20 à 35 g / L.

Une solution aqueuse contenant le substrat choisi pour la production des enzymes est ensuite ajoutée. Une composition enzymatique agissant sur la biomasse lignocellulosique produite par les champignons est enfin récupérée par filtration du milieu de culture. Dans cette composition, on retrouve notamment, la beta-glucosidase, l'endoglucanase et l'exoglucanase selon l'invention.

Dans un mode de réalisation, la solution aqueuse contenant le substrat choisi pour la production des enzymes est préparée à la concentration de 200-250 g / L. Cette solution contient en outre de préférence un substrat inducteur tel que le lactose. Cette solution aqueuse est injectée après l'épuisement du substrat carboné initial de façon à apporter une quantité optimisée, comprise entre 35 et 45 mg / g de cellules ("fed batch"). Pendant cette phase de "fed batch", la concentration résiduelle en sucre dans le milieu de culture est inférieure à 1 g / L et les enzymes agissant sur la biomasse lignocellulosique sont sécrétées par le champignon. Ces dernières peuvent être récupérées par filtration du milieu de culture.

L'invention a pour objet une composition enzymatique apte à agir sur la biomasse lignocellulosique, ladite composition enzymatique étant produite par des champignons filamenteux et comprenant au moins un polypeptide ayant une activité exoglucanase améliorée par rapport à l'activité exoglucanase de la protéine de référence CBH2. Par « champignons filamenteux », on entend notamment *Trichoderma,* plus préférentiellement *T. reesei.*

Enfin, l'invention a pour objet un procédé de production de biocarburant à partir de la biomasse comprenant les étapes successives suivantes :
- on met en suspension en phase aqueuse la biomasse à hydrolyser;
- on hydrolyse la biomasse lignocellulosique en présence d'une composition enzymatique telle que décrite précédemment de manière à produire un hydrolysat contenant du glucose;
- on fermente en présence d'un organisme fermentaire le glucose de l'hydrolysat de manière à produire un moût de fermentation;
- on sépare le biocarburant du moût de fermentation.

Dans un mode de réalisation, la biomasse à hydrolyser est mise en suspension en phase aqueuse à raison de 6 à 40 % de matière sèche, de préférence 20 à 30 %. Le pH est ajusté entre 4 et 5,5, de préférence entre 4,8 et 5,2 et la température entre 40°C et 60°C, de préférence entre 45°C et 50°C. La réaction d'hydrolyse est démarrée par l'ajout de la composition enzymatique agissant sur la biomasse lignocellulosique ; la quantité habituellement utilisée est de 10 à 30 mg de protéines excrétées par gramme de substrat prétraité ou moins. La réaction dure généralement de 15 à 48 heures. La réaction est suivie par dosage des sucres libérés, notamment le glucose. La solution de sucres est séparée de la fraction solide non hydrolysée, essentiellement constituée de lignine, par filtration ou centrifugation et ensuite traitée dans une unité de fermentation.

Après l'étape de fermentation, le biocarburant est séparé du moût de fermentation par exemple par distillation.

Un autre objet de l'invention est un procédé de production de biocarburant à partir de la biomasse, caractérisé en ce qu'il comprend les étapes successives suivantes :
- on met en suspension en phase aqueuse la biomasse à hydrolyser ;
- on ajoute simultanément à la suspension une composition enzymatique agissant sur la biomasse lignocellulosique telle que définie précédemment et un organisme fermentaire et on fermente le mélange de manière à produire un moût de fermentation ;
- on sépare le biocarburant du moût de fermentation.

De préférence, la composition enzymatique et l'organisme fermentaire sont ajoutés simultanément puis incubés à une température comprise entre 30°C et 35°C pour produire un moût de fermentation.

Selon ce mode de réalisation, la cellulose présente dans la biomasse est convertie en glucose, et en même temps, dans le même réacteur, l'organisme fermentaire (par exemple une levure) convertit le glucose en produit final selon un procédé de SSF (Simultaneous Saccharification and Fermentation) connu de l'homme du métier. Selon les capacités métaboliques et hydrolytiques de l'organisme fermentaire, le bon déroulement de l'opération peut nécessiter l'addition d'une quantité plus ou moins importante de mélange cellulolytique exogène.

Dans un autre mode de réalisation, l'organisme fermentaire produit le polypeptide objet de l'invention par sécrétion ou en surface de sa cellule, éventuellement conjointement à d'autres enzymes agissant sur la biomasse lignocellulosique, limitant ou supprimant ainsi le besoin en enzymes produites par le champignon filamenteux. De préférence, l'organisme fermentaire est une cellule hôte telle que décrite précédemment, selon l'invention.

De préférence, les cellules hôtes avec la composition enzymatique et/ou l'organisme fermentaire, sont ajoutés puis incubés à une température comprise entre 30°C et 35°C pour produire un moût de fermentation.

L'utilisation du polypeptide présentant une meilleure activité exoglucanase selon la présente invention présente ainsi l'avantage d'obtenir un meilleur rendement de production de glucose tout en employant moins d'enzyme qu'auparavant, ce qui présente également un avantage économique.

D'autres aspects, objets, avantages et caractéristiques de l'invention, seront présentés à la lecture de la description non restrictive qui suit et qui décrit des modes de réalisation préférés de l'invention donnés par le biais d'exemples et des figures.

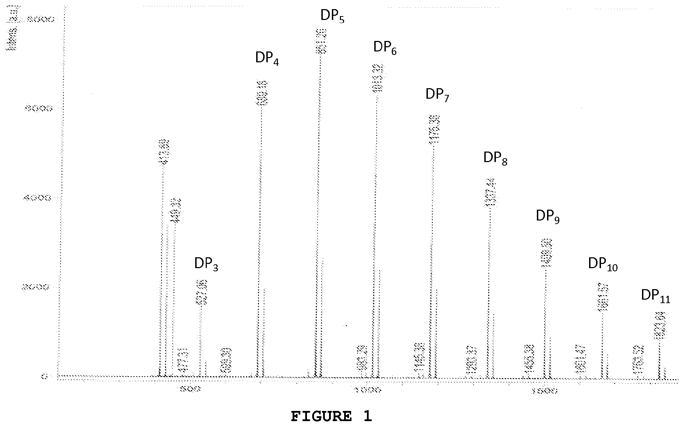

La Figure 1 est un spectre de masse MALDI-TOF représentant les cellodextrines DP3 à DP11 utilisées pour le criblage.

### EXEMPLES

### EXEMPLE 1 : Préparation de cellodextrines réduites (DP 3-11)

### 1-Hydrolyse de la cellulose (Adapté de Y-H. Percival Zhang, L. R. Lynd Analytical Biochemistry 322 (2003), 225-232.)

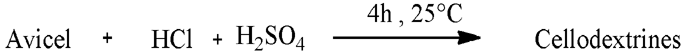

20 g de cellulose (Avicel. CAS Number 9004-34-6, Sigma-Aldrich Saint-Quentin Fallavier) sont ajoutés par portions et sous forte agitation à 160 mL d'une solution refroidie à 0°C d'acide chlorhydrique. De l'acide sulfurique, préalablement refroidi, est ajouté à la solution en plusieurs fois (4 x 10 mL). La réaction est maintenue sous agitation pendant 4 heures à 25°C avant d'être versée dans 1.8 L d'acétone refroidie à -20°C. Après 2 heures d'agitation, le précipité est filtré, repris dans 400 mL d'acétone refroidie puis filtré à nouveau. Le solide est alors repris dans 600 mL d'eau, puis agité pendant une nuit pour solubiliser les cellodextrines. Après filtration du solide, la fraction soluble contenant les cellodextrines est neutralisée avec 300 g de résine Amberlite IRA 400 OH^{-,} puis lyophilisée. Le lyophilisat est ensuite re-suspendu dans 500 mL de méthanol en présence d'ultra-sons pendant 30 minutes pour solubiliser les sucres de bas poids moléculaire avant d'être filtré puis lyophilisé à nouveau pour conduire à 6.8 g de cellodextrines de DP 3-11.

Pour le criblage, il a été choisi de travailler avec des substrats de plus haut poids moléculaire possible pour mimer au mieux la structure de la cellulose. Mais, les cellodextrines de haut poids moléculaire ne sont pas solubles, ceci empêchant une bonne reproductibilité des tests.

Une gamme de cellodextrines de DP 5-7 a donc été choisie, ce qui représente un bon compromis entre le haut poids moléculaire nécessaire et la solubilité des cellodextrines.

La Figure 1 présente un spectre de masse MALDI-TOF typiquement obtenu selon le procédé décrit ci-dessus.

La Figure 1 montre que les oligosaccharides isolés sont majoritairement de DP 5-7.

### 2-Réduction des cellodextrines

400 mg de borohydrure de sodium sont ajoutés à 2 g de cellodextrines DP 3-11 dilués dans 120 mL d'eau. Après 3 heures sous agitation à température ambiante, la solution est neutralisée par addition de résine Amberlite H+ IR 120, filtrée, puis lyophilisée, pour conduire à 2 g de cellodextrines réduites de manière quantitative. (C. Schou, G. Rasmussen, M-B. Kaltoft, B. Henrissat, M. Schulein Eur. J. Biochem. 217, 947-953 (1993)).

Un dosage au BCA (acide bicinchoninique) des cellodextrines isolées permet de vérifier la réduction totale des extrémités réductrices (Y.-H. Percival Zhang, L. R. Lynd Biomacromolecules 2005, 6, 1510-1515).

### EXEMPLE 2 : évolution par L-shuffling

La séquence du gène de la cellobiohydrolase 2 de *Trichoderma reesei* (SEQ ID NO :1) a été soumise à un tour de L-shuffling selon le procédé breveté décrit dans le brevet EP1104457 avec les gènes d'une exoglucanase putative de *Giberella zeae* PH-1 (SEQ ID NO :29) et d'une protéine hypothétique NECHADRAFT_73991 de *Nectria haematococca* mpVI (SEQ ID NO :31) présentant respectivement 63% et 69% d'homologie avec le gène parental CBH2 (SEQ ID NO :1). La séquence nucléique codant le peptide signal (SEQ ID NO :33) a été délétée lors du clonage, et remplacée par celle de la levure, de séquence SEQ ID NO :34 (séquence du peptide signal correspondant : SEQ ID NO :35).

### 1-Criblage à haut débit

Un test de criblage à haut débit a été mis au point afin de sélectionner les meilleurs clones issus du L-shuffling, c'est-à-dire ceux présentant au moins 20% d'amélioration de l'activité cellobiohydrolase par rapport à l'enzyme de référence CBH2 (SEQ ID NO :2).

Le test de criblage à haut débit a été réalisé selon les étapes suivantes :
- isolement sur gélose des clones de *Y. lipolytica* exprimant les variants de L-shuffling de l'enzyme selon l'invention et mise en pré-cultures en milieu YNBcasa (yeast nitrogen base 1.7 g / L, NH₄Cl 10 g / L, glucose 10 g / L, casamino acids 2 g / L, pH7) desdites colonies pendant 36 heures à 28°C ;
- inoculation d'un milieu YTD (extrait de levure 10 g / L, tryptone 20 g / L, glucose 2.5 g / L, pH 6.8) additionné de tétracycline à 12.5 µg / mL à 5% avec la pré-culture puis incubation 20 heures à 28°C ;
- inoculation du milieu d'expression contenant l'inducteur (acide oléique) à raison de 20 g / L à 10% avec la culture précédente puis incubation 96 heures à 28°C ;
- centrifugation 5 minutes à 1500 rpm ;
- prélèvement de 100 µL de surnageant ;
- ajout de 100 µL de CD réduites à 1 g / L dans du tampon citrate phosphate 0.1 M à pH 6 ;
- incubation 24 heures à 35°C ;
- centrifugation pendant 5 minutes à 2500 rpm ;
- prélèvement de 80 µL de surnageant ;
- ajout de 80 µL de réactif DNS ;
- incubation 12 minutes à 105°C puis 5 minutes sur la glace ;
- lecture de la densité optique (DO) à 540 nm sur 120 µL.

Dans ces conditions de criblage, une amélioration de l'activité cellobiohydrolase (augmentation de la DO à 540 nm) par rapport à l'enzyme de référence CBH2 (SEQ ID NO :2) a été trouvée dans plusieurs clones. Parmi ces clones, on peut citer les clones 35B7 , 95B7, 100F11, 139F12, 157B11, 161A1, 161C12, 189H8, 196D9, 198E11, 251B4, 251C4 et 382A2, codant respectivement pour les enzymes SEQ ID NO :4, SEQ ID NO :6, SEQ ID NO :8, SEQ ID NO :10, SEQ ID NO :12, SEQ ID NO :14, SEQ ID NO :16, SEQ ID NO :18, SEQ ID NO :20, SEQ ID NO :22, SEQ ID NO :24, SEQ ID NO :26 et SEQ ID NO :28.

### 2-Détermination de l'amélioration de l'activité cellobiohydrolase

### 2-1/ Sur le substrat cellodextrines réduites

Afin d'estimer le kcat relatif des variants sélectionnés au premier tour de L-shuffling par rapport à l'enzyme de référence CBH2 (SEQ ID NO :2), on procède de la façon suivante :
- préparation d'une culture stock de *Y. lipolytica* exprimant une enzyme recombinante selon l'invention pendant toute la nuit à 28°C ;
- ensemencement d'un milieu d'expression avec un volume de culture stock permettant d'avoir une densité optique à 600 nm égale à 0.2 au début de la culture ;
- culture desdites cellules à 28°C pendant 96 heures ;
- centrifugation à 8000 rpm pendant 5 minutes ;
- incubation de 100 µL de surnageant avec 100 µL de tampon citrate phosphate 0.1 M pH 6 contenant 1 % de CD réduites pendant 4 heures à 35°C et 50°C ;
- prélèvement de 100 µL de réaction ;
- ajout de 100 µL de réactif DNS ;
- incubation 5 minutes à 100°C ;
- incubation 3 minutes sur la glace ;
- centrifugation 10 minutes à 3000 rpm ;
- lecture de la densité optique à 540 nm sur 150 µL.

Selon l'invention, le calcul des kcat est fait de la façon suivante :
- tracé de la courbe des DO à 540 nm en fonction du volume de surnageant de culture dans le test ;
- soustraction de la valeur du témoin négatif ;
- division par le coefficient de la gamme étalon de glucose (différentes quantités de glucose sont révélées avec le DNS) ;
- division par le temps de réaction (240 minutes).

Le tableau 2 présente la valeur des kcat ainsi que les facteurs d'amélioration obtenus pour les clones 35B7, 95B7, 100F11, 139F12, 157B11, 161A1, 161C12, 189H8, 196D9, 198E11, 251B4, 251C4 et 382A2 codant respectivement les enzymes SEQ ID NO :4, SEQ ID NO :6, SEQ ID NO :8, SEQ ID NO :10, SEQ ID NO :12, SEQ ID NO :14, SEQ ID NO :16, SEQ ID NO :18, SEQ ID NO :20, SEQ ID NO :22, SEQ ID NO :24, SEQ ID NO :26 et SEQ ID NO :28 par rapport à la protéine de référence CBH2 (SEQ ID NO :2) dans ces conditions expérimentales.

**TABLEAU 2 : amélioration de l'activité cellobiohydrolase sur CD réduites**

| | | **35°C** | | **50°C** | |
|---|---|---|---|---|---|
| | **Clone** | **Kcat (min⁻¹)** | **Facteur amélioration** | **Kcat (min⁻¹)** | **Facteur amélioration** |
| **Clones du premier tour** | **35B7** | 0,166 | 3,8 | 0,2345 | 1,6 |
| | **95B7** | 0,287 | 6,6 | 0,715 | 4,8 |
| | **100F11** | 0,0508 | 1,2 | 0,1375 | 0,9 |
| | **139F12** | 0,1719 | 3,9 | 0,2328 | 1,6 |
| | **157B11** | 0,113 | 2,6 | 0,2061 | 1,4 |
| | **161A1** | 0,0577 | 1,3 | 0,1175 | 0,8 |
| | **161C12** | 0,1086 | 2,5 | 0,2162 | 1,4 |
| | **189H8** | 0,0872 | 2,0 | 0,1792 | 1,2 |
| | **196D9** | 0,1055 | 2,4 | 0,1969 | 1,3 |
| | **198E11** | 0,1218 | 2,8 | 0,1757 | 1,2 |
| | **251B4** | 0,0495 | 1,1 | 0,0865 | 0,6 |
| | **251C4** | 0,0623 | 1,4 | 0,1315 | 0,9 |
| | **382A2** | 0,315 | 7,2 | 0,552 | 3,7 |
| **Protéine de référence** | **cbh2** | 0,0436 | 1 | 0,1501 | 1 |

Les résultats montrent une amélioration d'activité enzymatique par rapport à l'enzyme de référence CBH2 (SEQ ID No :2) pour les clones 35B7, 95B7, 100F11, 139F12, 157B11, 161A1, 161C12, 189H8, 196D9, 198E11, 251B4, 251C4 et 382A2 codant respectivement pour les enzymes SEQ ID NO :4, SEQ ID NO :6, SEQ ID NO :8, SEQ ID NO :10, SEQ ID NO :12, SEQ ID NO :14, SEQ ID NO :16, SEQ ID NO :18, SEQ ID NO :20, SEQ ID NO :22, SEQ ID NO :24, SEQ ID NO :26 et SEQ ID NO :28 que ce soit à 35°C ou à 50°C.

### 2-2/ Sur le substrat Avicel

L'amélioration d'activité des clones 35B7, 95B7, 100F11, 139F12, 157B11, 161A1, 161C12, 189H8, 196D9, 198E11, 251B4, 251C4 et 382A2 codant respectivement pour les enzymes SEQ ID NO :4, SEQ ID NO :6, SEQ ID NO :8, SEQ ID NO :10, SEQ ID NO :12, SEQ ID NO :14, SEQ ID NO :16, SEQ ID NO :18, SEQ ID NO :20, SEQ ID NO :22, SEQ ID NO :24, SEQ ID NO :26 et SEQ ID NO :28 a ensuite été confirmée sur un second substrat : Avicel.

La détermination de l'amélioration de l'activité sur ce substrat est effectuée en mesure en point final selon le protocole suivant :
- préparation d'une culture stock de *Y. lipolytica* exprimant une enzyme recombinante selon l'invention pendant toute la nuit à 28°C ;
- ensemencement d'un milieu d'expression avec un volume de culture stock permettant d'avoir une densité optique à 600 nm égale à 0.2 au début de la culture ;
- culture desdites cellules à 28°C pendant 96 heures ;
- centrifugation à 8000 rpm pendant 5 minutes ;
- incubation de 100 µL de surnageant avec 100 µL de tampon citrate phosphate 0.1 M pH 6 contenant 1 % d'Avicel pendant 18 heures à 35 et 50°C ;
- prélèvement de 100 µL de réaction ;
- ajout de 100 µL de réactif DNS ;
- incubation 5 minutes à 100°C ;
- incubation 3 minutes sur la glace ;
- centrifugation 10 minutes à 3000 rpm ;
- lecture de la densité optique à 540 nm sur 150 µL.

Le tableau 3 présente la valeur des DO à 540 nm (après soustraction de la valeur du témoin négatif) ainsi que le facteur d'amélioration obtenus pour les clones 35B7, 95B7, 100F11, 139F12, 157B11, 161A1, 161C12, 189H8, 196D9, 198E11, 251B4, 251C4 et 382A2 dans ces conditions expérimentales.

**TABLEAU 3 : amélioration de l'activité cellobiohydrolase sur Avicel**

| | | **35°C** | | **50°C** | |
|---|---|---|---|---|---|
| | **Clone** | **Delta DO 540 nm** | **Facteur amélioration** | **Delta DO 540 nm** | **Facteur amélioration** |
| **Clones du premier tour** | **35B7** | 0,0617 | 1,0 | 0,0948 | 0,8 |
| | **95B7** | 0,0396 | 0,6 | 0,0555 | 0,4 |
| | **100F11** | 0,038 | 0,6 | 0,0159 | 0,1 |
| | **139F12** | 0,06 | 0,9 | 0,0365 | 0,3 |
| | **157B11** | 0,0456 | 0,7 | 0,0319 | 0,3 |
| | **161A1** | 0,0508 | 0,8 | 0,0237 | 0,2 |
| | **161C12** | 0,0564 | 0,9 | 0,0595 | 0,5 |
| | **189H8** | 0,0676 | 1,0 | 0,0573 | 0,5 |
| | **196D9** | 0,0565 | 0,9 | 0,0874 | 0,7 |
| | **198E11** | 0,0867 | 1,3 | 0,0546 | 0,4 |
| | **251B4** | 0,0765 | 1,2 | 0,0622 | 0,5 |
| | **251C4** | 0,063 | 1,0 | 0,0889 | 0,7 |
| | **382A2** | 0,2476 | 3,8 | 0,2256 | 1,8 |
| **Protéine de référence** | **cbh2** | 0,0644 | 1 | 0,1252 | 1 |

Les résultats du tableau 3 montrent une amélioration de l'activité enzymatique par rapport à l'enzyme de référence CBH2 (SEQ ID No :2) à 35°C pour les clones 198E11 et 251B4 (respectivement SEQ ID No :22 et 24) ainsi qu'une amélioration de l'activité enzymatique par rapport à l'enzyme de référence CBH2 (SEQ ID No :2) à 35°C et à 50°C pour le clone 382A2 (SEQ ID No :28).

## Revendications

1. Polypeptide isolé ou purifié **caractérisé en ce qu'**il a une activité exoglucanase améliorée d'au moins 10% à une température d'environ 35°C et/ou d'environ 50°C par rapport à l'activité exoglucanase de la protéine de référence CBH2 de séquence SEQ ID NO :2, ledit polypeptide étant choisi dans le groupe consistant en :
i. une séquence d'acides aminés choisie parmi SEQ ID NO : 28, SEQ ID NO : 6, SEQ ID NO : 4, SEQ ID NO : 8, SEQ ID NO : 10, SEQ ID NO : 12, SEQ ID NO : 14, SEQ ID NO : 16, SEQ ID NO : 18, SEQ ID NO : 20, SEQ ID NO : 22, SEQ ID NO : 24 et SEQ ID NO : 26; et
ii. une séquence d'acides aminés présentant, par rapport à la séquence SEQ ID NO : 28, SEQ ID NO : 6, SEQ ID NO : 4, SEQ ID NO : 8, SEQ ID NO : 10, SEQ ID NO : 12, SEQ ID NO : 14, SEQ ID NO : 16, SEQ ID NO : 18, SEQ ID NO : 20, SEQ ID NO : 22, SEQ ID NO : 24 et SEQ ID NO : 26, un pourcentage d'identité d'au moins 99%.

2. Acide nucléique purifié ou isolé, **caractérisé en ce qu'**il code au moins un polypeptide selon la revendication **1.**

3. Acide nucléique purifié ou isolé selon la revendication **2** choisi parmi les séquences suivantes : SEQ ID NO : 27, SEQ ID NO : 5, SEQ ID NO : 3,, SEQ ID NO : 7, SEQ ID NO : 9, SEQ ID NO : 11; SEQ ID NO : 13, SEQ ID NO : 15, SEQ ID NO : 17 et SEQ ID NO : 19, SEQ ID NO : 21, SEQ ID NO : 23 et SEQ ID NO : 25.

4. Vecteur **caractérisé en ce qu'**il comprend au moins un acide nucléique selon l'une des revendications **2 ou 3.**

5. Cellule hôte isolée **caractérisée en ce qu'**elle comprend au moins un polypeptide selon la revendication **1,** ou au moins un acide nucléique selon l'une des revendications **2 ou 3,** ou au moins un vecteur selon la revendication **4.**

6. Cellule hôte isolée selon la revendication **5, caractérisée en ce qu'**elle est choisie parmi *Trichoderma, Aspergillus, Neurospora, Humicola, Penicillium, Fusarium, Thermomonospora, Myceliophthora, Chrysosporium, Bacillus, Pseudomonas, Escherichia, Clostridium, Cellulomonas, Streptomyces, Yarrowia, Pichia* et *Saccharomyces.*

7. Cellule hôte isolée selon l'une des revendications **5 ou 6, caractérisée en ce qu'**elle est choisie parmi *Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisae, Myceliophthora thermopila, Chrysosporium lucknowense, Penicillium pinophilum, Penicillium oxalicum, Escherichia coli, Clostridium acetobutylicum, Clostridium saccharolyticum, Clostridium benjerinckii, Clostridium butylicum, Pichia pastoris, Yarrowia lipolityca* et *Saccharomyces cerevisiae.*

8. Utilisation dudit polypeptide selon la revendication **1** pour l'hydrolyse de la cellulose.

9. Utilisation dudit polypeptide selon la revendication **1** pour la production de biocarburant.

10. Composition enzymatique apte à agir sur la biomasse lignocellulosique, ladite composition enzymatique étant produite par des champignons filamenteux et comprenant au moins un polypeptide selon la revendication **1.**

11. Procédé de production de biocarburant à partir de la biomasse lignocellulosique, **caractérisé en ce qu'**il comprend les étapes successives suivantes :
- on met en suspension en phase aqueuse la biomasse à hydrolyser ;
- on hydrolyse la biomasse en présence d'une composition enzymatique selon la revendication **10** la biomasse lignocellulosique de manière à produire un hydrolysat contenant du glucose ;
- on fermente en présence d'un organisme fermentaire le glucose de l'hydrolysat de manière à produire un moût de fermentation ;
- on sépare le biocarburant du moût de fermentation.

12. Procédé de production de biocarburant à partir de la biomasse, **caractérisé en ce qu'**il comprend les étapes successives suivantes :
- on met en suspension en phase aqueuse la biomasse à hydrolyser ;
- on ajoute simultanément à la suspension une composition enzymatique selon la revendication **10** et un organisme fermentaire et on fermente le mélange de manière à produire un moût de fermentation ;
- on sépare le biocarburant du moût de fermentation.

13. Procédé selon l'une des revendications **11 ou 12,** dans lequel l'organisme fermentaire est une cellule hôte selon l'une des revendications **5 à 7.**

## Patentansprüche

1. Isoliertes oder gereinigtes Polypeptid, **dadurch gekennzeichnet, dass** es eine um wenigstens 10% erhöhte Exoglucanase-Aktivität bei einer Temperatur von ungefähr 35°C und/oder ungefähr 50°C bezogen auf die Exoglucanase-Aktivität des Referenzproteins CBH2 der Sequenz SEQ ID NO:2 aufweist, wobei das Polypeptid gewählt ist aus der Gruppe, umfassend:
i. eine Aminosäuresequenz, gewählt aus SEQ ID NO: 28, SEQ ID NO: 6, SEQ ID NO: 4, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24 und SEQ ID NO: 26; und
ii. eine Aminosäuresequenz, die bezogen auf die Sequenz SEQ ID NO: 28, SEQ ID NO: 6, SEQ ID NO: 4, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24 und SEQ ID NO: 26, eine prozentuale Identität von wenigstens 99 % aufweist.

2. Gereinigte oder isolierte Nukleinsäure, **dadurch gekennzeichnet, dass** sie für wenigstens ein Polypeptid nach Anspruch 1 kodiert.

3. Gereinigte oder isolierte Nukleinsäure nach Anspruch **2,** gewählt aus den folgenden Sequenzen: SEQ ID NO: 27, SEQ ID NO: 5, SEQ ID NO: 3, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 und SEQ ID NO: 25.

4. Vektor, **dadurch gekennzeichnet, dass** er wenigstens eine Nukleinsäure nach einem der Ansprüche **2 oder 3** umfasst.

5. Isolierte Wirtszelle, **dadurch gekennzeichnet, dass** sie wenigstens ein Polypeptid nach Anspruch **1,** oder wenigstens eine Nukleinsäure nach einem der Ansprüche **2 oder 3,** oder wenigstens einen Vektor nach Anspruch **4** umfasst.

6. Isolierte Wirtszelle nach Anspruch **5, dadurch gekennzeichnet, dass** sie gewählt ist aus *Trichoderma, Aspergillus, Neurospora, Humicola, Penicillium, Fusarium, Thermomonospora, Myceliophthora, Chrysosporium, Bacillus, Pseudomonas, Escherichia, Clostridium, Cellulomonas, Streptomyces, Yarrowia, Pichia und Saccharomyces.*

7. Isolierte Wirtszelle nach einem der Ansprüche **5 oder 6, dadurch gekennzeichnet, dass** sie gewählt ist aus *Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisae, Myceliophthora thermopila, Chrysosporium lucknowense, Penicillium pinophilum, Penicillium oxalicum, Escherichia coli, Clostridium acetobutylicum, Clostridium saccharolyticum, Clostridium benjerinckii, Clostridium butylicum, Pichia pastoris, Yarrowia lipolityca und Saccharomyces cerevisiae.*

8. Verwendung des Polypeptids nach Anspruch **1** für die Hydrolyse von Cellulose.

9. Verwendung des Polypeptids nach Anspruch **1** zur Herstellung von Biokraftstoff.

10. Enzymatische Zusammensetzung, die in der Lage ist, auf lignozellulosehaltige Biomasse einzuwirken, wobei die enzymatische Zusammensetzung durch filamentöse Pilze hergestellt wird und wenigstens ein Polypeptid nach Anspruch **1** umfasst.

11. Verfahren zur Herstellung von Biokraftstoff aus lignocellulosehaltiger Biomasse, **dadurch gekennzeichnet, dass** es die folgenden aufeinanderfolgenden Schritte umfasst:
- Suspendieren der zu hydrolysierenden Biomasse in einer wässrigen Phase;
- Hydrolysieren der Biomasse in Gegenwart einer enzymatischen Zusammensetzung nach Anspruch **10,** um ein Hydrolysat zu erzeugen, das Glucose enthält;
- Fermentieren der Glucose des Hydrolysats in Gegenwart eines fermentativen Organismus, um eine Fermentationsmaische herzustellen;
- Abtrennen des Biokraftstoffs von der Fermentationsmaische.

12. Verfahren zur Herstellung von Biokraftstoff aus Biomasse, **dadurch gekennzeichnet, dass** es die folgenden aufeinanderfolgenden Schritte umfasst:
- Suspendieren der zu hydrolysierenden Biomasse in einer wässrigen Phase;
- Gleichzeitiges Hinzufügen einer enzymatischen Zusammensetzung nach Anspruch **10** und eines fermentierenden Organismus zu der Suspension und die Fermentieren der Mischung, um eine Fermentationsmaische herzustellen;
- Abtrennen des Biokraftstoffs von der Fermentationsmaische.

13. Verfahren nach einem der Ansprüche **11 oder 12,** wobei der fermentative Organismus eine Wirtszelle nach einem der Ansprüche **5 bis 7** ist.

## Claims

1. Isolated or purified polypeptide **characterised in that** it has an at least 10% enhanced exoglucanase activity at a temperature of about 35°C and/or about 50°C with respect to the exoglucanase activity of the reference protein CBH2 of sequence SEQ ID NO: 2, said polypeptide being selected from the group consisting of:
i. an amino acid sequence selected from SEQ ID NO: 28, SEQ ID NO: 6, SEQ ID NO: 4, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24 and SEQ ID NO: 26; and
ii. an amino acid sequence having, with respect to the sequence SEQ ID NO: 28, SEQ ID NO: 6, SEQ ID NO: 4, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24 and SEQ ID NO: 26, a percent identity of at least 99%.

2. Purified or isolated nucleic acid, **characterised in that** it encodes at least one polypeptide according to claim 1.

3. Purified or isolated nucleic acid according to claim 2 selected from the following sequences: SEQ ID NO: 27, SEQ ID NO: 5, SEQ ID NO: 3, SEQ ID NO: 7, SEQ ID NO: 9, SEQ ID NO: 11; SEQ ID NO: 13, SEQ ID NO: 15, SEQ ID NO: 17 and SEQ ID NO: 19, SEQ ID NO: 21, SEQ ID NO: 23 and SEQ ID NO: 25.

4. Vector **characterised in that** it comprises at least one nucleic acid according to one of claims 2 or 3.

5. Isolated host cell **characterised in that** it comprises at least one polypeptide according to claim 1, or at least one nucleic acid according to one of claims 2 or 3, or at least one vector according to claim 4.

6. Isolated host cell according to claim 5, **characterised in that** it is selected from *Trichoderma, Aspergillus, Neurospora, Humicola, Penicillium, Fusarium, Thermomonospora, Myceliophthora, Chrysosporium, Bacillus, Pseudomonas, Escherichia, Clostridium, Cellulomonas, Streptomyces, Yarrowia, Pichia* and *Saccharomyces.*

7. Isolated host cell according to one of claims 5 or 6, **characterised in that** it is selected from *Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisae, Myceliophthora thermopila, Chrysosporium lucknowense, Penicillium pinophilum, Penicillium oxalicum, Escherichia coli, Clostridium acetobutylicum, Clostridium saccharolyticum, Clostridium benjerinckii, Clostridium butylicum, Pichia pastoris, Yarrowia lipolityca* and *Saccharomyces cerevisiae.*

8. Use of said polypeptide according to claim 1 for hydrolysing cellulose.

9. Use of said polypeptide according to claim 1 for producing biofuel.

10. Enzyme composition capable of acting upon lignocellulosic biomass, said enzyme composition being produced by filamentous fungi and comprising at least one polypeptide according to claim 1.

11. Method for producing biofuel from lignocellulosic biomass, **characterised in that** it comprises the following successive steps:
- suspending the biomass to be hydrolysed in aqueous phase;
- hydrolysing the biomass in the presence of an enzyme composition according to claim 10 the lignocellulosic biomass so as to produce a hydrolysate containing glucose;
- fermenting in the presence of a fermentative organism the glucose from the hydrolysate so as to produce a fermentation wort;
- separating the biofuel from the fermentation wort.

12. Method for producing biofuel from biomass, **characterised in that** it comprises the following successive steps:
- suspending the biomass to be hydrolysed in aqueous phase;
- simultaneously adding to the suspension an enzyme composition according to claim 10 and a fermentative organism and fermenting the mixture so as to produce a fermentation wort;
- separating the biofuel from the fermentation wort.

13. Method according to one of claims 11 or 12, wherein the fermentative organism is a host cell according to one of claims 5 to 7.


### SEQUENCE LISTING

<110> IFP Energies Nouvelles et al
<120> VARIANTS D'EXOGLUCANASES A ACTIVITE AMELIOREE ET LEURS UTILISATIONS
<130> BFF140338
<160> 35
<170> PatentIn version 3.5
<210> 1
   <211> 1413
   <212> DNA
   <213> Trichoderma reesei
<400> 1
   atgattgtcg gcattctcac cacgctggct acgctggcca cactcgcagc tagtgtgcct      60
   ctagaggagc ggcaagcttg ctcaagcgtc tggggccaat gtggtggcca gaattggtcg     120
   ggtccgactt gctgtgcttc cggaagcaca tgcgtctact ccaacgacta ttactcccag     180
   tgtcttcccg gcgctgcaag ctcaagctcg tccacgcgcg ccgcgtcgac gacttctcga     240
   gtatccccca caacatcccg gtcgagctcc gcgacgcctc cacctggttc tactactacc     300
   agagtacctc cagtcggatc gggaaccgct acgtattcag gcaacccttt tgttggggtc     360
   actccttggg ccaatgcata ttacgcctct gaagttagca gcctcgctat tcctagcttg     420
   actggagcca tggccactgc tgcagcagct gtcgcaaagg ttccctcttt tatgtggcta     480
   gatactcttg acaagacccc tctcatggag caaaccttgg ccgacatccg caccgccaac     540
   aagaatggcg gtaactatgc cggacagttt gtggtgtatg acttgccgga tcgcgattgc     600
   gctgcccttg cctcgaatgg cgaatactct attgccgatg gtggcgtcgc caaatataag     660
   aactatatcg acaccattcg tcaaattgtc gtggaatatt ccgatatccg gaccctcctg     720
   gttattgagc ctgactctct tgccaacctg gtgaccaacc tcggtactcc aaagtgtgcc     780
   aatgctcagt cagcctacct tgagtgcatc aactacgccg tcacacagct gaaccttcca     840
   aatgttgcga tgtatttgga cgctggccat gcaggatggc ttggctggcc ggcaaaccaa     900
   gacccggccg ctcagctatt tgcaaatgtt tacaagaatg catcgtctcc gagagctctt     960
   cgcggattgg caaccaatgt cgccaactac aacgggtgga acattaccag ccccccatcg    1020
   tacacgcaag gcaacgctgt ctacaacgag aagctgtaca tccacgctat tggacctctt    1080
   cttgccaatc acggctggtc caacgccttc ttcatcactg atcaaggtcg atcgggaaag    1140
   cagcctaccg gacagcaaca gtggggagac tggtgcaatg tgatcggcac cggatttggt    1200
   attcgcccat ccgcaaacac tggggactcg ttgctggatt cgtttgtctg ggtcaagcca    1260
   ggcggcgagt gtgacggcac cagcgacagc agtgcgccac gatttgactc ccactgtgcg    1320
   ctcccagatg ccttgcaacc ggcgcctcaa gctggtgctt ggttccaagc ctactttgtg    1380
   cagcttctca caaacgcaaa cccatcgttc ctg                                  1413
<210> 2
   <211> 471
   <212> PRT
   <213> Trichoderma reesei
<400> 2
   Met Ile Val Gly Ile Leu Thr Thr Leu Ala Thr Leu Ala Thr Leu Ala
   1               5                   10                  15
   Ala Ser Val Pro Leu Glu Glu Arg Gln Ala Cys Ser Ser Val Trp Gly
               20                  25                  30
   Gln Cys Gly Gly Gln Asn Trp Ser Gly Pro Thr Cys Cys Ala Ser Gly
           35                  40                  45
   Ser Thr Cys Val Tyr Ser Asn Asp Tyr Tyr Ser Gln Cys Leu Pro Gly
       50                  55                  60
   Ala Ala Ser Ser Ser Ser Ser Thr Arg Ala Ala Ser Thr Thr Ser Arg
   65                  70                  75                  80
   Val Ser Pro Thr Thr Ser Arg Ser Ser Ser Ala Thr Pro Pro Pro Gly
                   85                  90                  95
   Ser Thr Thr Thr Arg Val Pro Pro Val Gly Ser Gly Thr Ala Thr Tyr
               100                 105                 110
   Ser Gly Asn Pro Phe Val Gly Val Thr Pro Trp Ala Asn Ala Tyr Tyr
           115                 120                 125
   Ala Ser Glu Val Ser Ser Leu Ala Ile Pro Ser Leu Thr Gly Ala Met
       130                 135                 140
   Ala Thr Ala Ala Ala Ala Val Ala Lys Val Pro Ser Phe Met Trp Leu
   145                 150                 155                 160
   Asp Thr Leu Asp Lys Thr Pro Leu Met Glu Gln Thr Leu Ala Asp Ile
                   165                 170                 175
   Arg Thr Ala Asn Lys Asn Gly Gly Asn Tyr Ala Gly Gln Phe Val Val
               180                 185                 190
   Tyr Asp Leu Pro Asp Arg Asp Cys Ala Ala Leu Ala Ser Asn Gly Glu
           195                 200                 205
   Tyr Ser Ile Ala Asp Gly Gly Val Ala Lys Tyr Lys Asn Tyr Ile Asp
       210                 215                 220
   Thr Ile Arg Gln Ile Val Val Glu Tyr Ser Asp Ile Arg Thr Leu Leu
   225                 230                 235                 240
   Val Ile Glu Pro Asp Ser Leu Ala Asn Leu Val Thr Asn Leu Gly Thr
                   245                 250                 255
   Pro Lys Cys Ala Asn Ala Gln Ser Ala Tyr Leu Glu Cys Ile Asn Tyr
               260                 265                 270
   Ala Val Thr Gln Leu Asn Leu Pro Asn Val Ala Met Tyr Leu Asp Ala
           275                 280                 285
   Gly His Ala Gly Trp Leu Gly Trp Pro Ala Asn Gln Asp Pro Ala Ala
       290                 295                 300
   Gln Leu Phe Ala Asn Val Tyr Lys Asn Ala Ser Ser Pro Arg Ala Leu
   305                 310                 315                 320
   Arg Gly Leu Ala Thr Asn Val Ala Asn Tyr Asn Gly Trp Asn Ile Thr
                   325                 330                 335
   Ser Pro Pro Ser Tyr Thr Gln Gly Asn Ala Val Tyr Asn Glu Lys Leu
               340                 345                 350
   Tyr Ile His Ala Ile Gly Pro Leu Leu Ala Asn His Gly Trp Ser Asn
           355                 360                 365
   Ala Phe Phe Ile Thr Asp Gln Gly Arg Ser Gly Lys Gln Pro Thr Gly
       370                 375                 380
   Gln Gln Gln Trp Gly Asp Trp Cys Asn Val Ile Gly Thr Gly Phe Gly
   385                 390                 395                 400
   Ile Arg Pro Ser Ala Asn Thr Gly Asp Ser Leu Leu Asp Ser Phe Val
                   405                 410                 415
   Trp Val Lys Pro Gly Gly Glu Cys Asp Gly Thr Ser Asp Ser Ser Ala
               420                 425                 430
   Pro Arg Phe Asp Ser His Cys Ala Leu Pro Asp Ala Leu Gln Pro Ala
           435                 440                 445
   Pro Gln Ala Gly Ala Trp Phe Gln Ala Tyr Phe Val Gln Leu Leu Thr
       450                 455                 460
   Asn Ala Asn Pro Ser Phe Leu
   465 470
<210> 3
   <211> 1353
   <212> DNA
   <213> Artificial Sequence
<220>
   <223> Clone 35B7
<400> 3
   gcccctcttg ttgaggagcg ccaggcttgc gctgcccaat gggcccagtg tggtggcttc 60
   agctggaatg gtgctacctg ctgccagtct ggtagctact gcagcaagat caacgactat 120
   tactctcagt gcattcctgg agaaggcccc gccacttcca agtcgagcac tcttcctgct 180
   tccaccacaa caactcagcc aacttccact tcgactgctg gaacctcttc cactaccaag 240
   cctcctccag ctggatcggg aaccgctacg tattcaggca acccttactc tggggtcaac 300
   ctttgggcca atagctatta ccgctctgaa gttaccaacc tcgctattcc taagttgagc 360
   ggagccatgg ccactgctgc agcaaaggtc gcagatgttc cctcttttca gtggatggat 420
   tctttcgacc acatctccct catggaggac accttggtcg acatccgcaa ggccaacctg 480
   gctggcggta actatgccgg acagtttgtg gtgtatgact tgccggatcg cgattgcgct 540
   gccgccgcct cgaatggcga atactctctt gccgatggtg gcgtcgccaa atataaggcc 600
   tatatcgcca agattaaggg tattctccag gactattccg atacccggat cattctggtt 660
   attgagcctg actctcttgc caacctggtg accaacatga acgtcccaaa gtgtgccaat 720
   gctgcttcag cctacaagga gctcaccatt cacgccctca aggagctgaa ccttccaaat 780
   gtttccatgt atatcgacgc tggccatggt ggatggcttg gctggccggc aaaccaaggc 840
   ccggccgcta aactatttgc aagcatctac aaggatgcag gcaagccggc cgctcttcgc 900
   ggattggcaa ccaatgtcgc caactacaac gcctggagcc tcagcagcgc tccaccttac 960
   acgcaaggcg cctctatcta cgacgagaag agcttcatcc acgctatggg acctcttctt 1020
   gagcagaatg gctggcctgg tgcccacttc atcactgatc aaggtcgatc gggaaagcag 1080
   cctaccggac agatccagtg gggagactgg tgcaattcca agggcaccgg atttggtatt 1140
   cgcccatccg caaacactgg ggactcgttg ctggatgctt ttgtctgggt caagccaggc 1200
   ggcgagtctg acggcaccag cgacaccagt gcggctcgat acgactacca ctgtggtatt 1260
   gacggcgccg tcaagccggc gcctgaggct ggtacctggt tccaagccta ctttgagcag 1320
   cttctcacaa acgcaaaccc atcgttcctg taa 1353
<210> 4
   <211> 450
   <212> PRT
   <213> Artificial Sequence
<220>
   <223> Clone 35B7
<400> 4
   Ala Pro Leu Val Glu Glu Arg Gln Ala Cys Ala Ala Gln Trp Ala Gln
   1               5                   10                  15
   Cys Gly Gly Phe Ser Trp Asn Gly Ala Thr Cys Cys Gln Ser Gly Ser
               20                  25                  30
   Tyr Cys Ser Lys Ile Asn Asp Tyr Tyr Ser Gln Cys Ile Pro Gly Glu
           35                  40                  45
   Gly Pro Ala Thr Ser Lys Ser Ser Thr Leu Pro Ala Ser Thr Thr Thr
       50                  55                  60
   Thr Gln Pro Thr Ser Thr Ser Thr Ala Gly Thr Ser Ser Thr Thr Lys
   65                  70                  75                  80
   Pro Pro Pro Ala Gly Ser Gly Thr Ala Thr Tyr Ser Gly Asn Pro Tyr
                   85                  90                  95
   Ser Gly Val Asn Leu Trp Ala Asn Ser Tyr Tyr Arg Ser Glu Val Thr
               100                 105                 110
   Asn Leu Ala Ile Pro Lys Leu Ser Gly Ala Met Ala Thr Ala Ala Ala
           115                 120                 125
   Lys Val Ala Asp Val Pro Ser Phe Gln Trp Met Asp Ser Phe Asp His
       130                 135                 140
   Ile Ser Leu Met Glu Asp Thr Leu Val Asp Ile Arg Lys Ala Asn Leu
   145                 150                 155                 160
   Ala Gly Gly Asn Tyr Ala Gly Gln Phe Val Val Tyr Asp Leu Pro Asp
                   165                 170                 175
   Arg Asp Cys Ala Ala Ala Ala Ser Asn Gly Glu Tyr Ser Leu Ala Asp
               180                 185                 190
   Gly Gly Val Ala Lys Tyr Lys Ala Tyr Ile Ala Lys Ile Lys Gly Ile
           195                 200                 205
   Leu Gln Asp Tyr Ser Asp Thr Arg Ile Ile Leu Val Ile Glu Pro Asp
       210                 215                 220
   Ser Leu Ala Asn Leu Val Thr Asn Met Asn Val Pro Lys Cys Ala Asn
   225                 230                 235                 240
   Ala Ala Ser Ala Tyr Lys Glu Leu Thr Ile His Ala Leu Lys Glu Leu
                   245                 250                 255
   Asn Leu Pro Asn Val Ser Met Tyr Ile Asp Ala Gly His Gly Gly Trp
               260                 265                 270
   Leu Gly Trp Pro Ala Asn Gln Gly Pro Ala Ala Lys Leu Phe Ala Ser
           275                 280                 285
   Ile Tyr Lys Asp Ala Gly Lys Pro Ala Ala Leu Arg Gly Leu Ala Thr
       290                 295                 300
   Asn Val Ala Asn Tyr Asn Ala Trp Ser Leu Ser Ser Ala Pro Pro Tyr
   305                 310                 315                 320
   Thr Gln Gly Ala Ser Ile Tyr Asp Glu Lys Ser Phe Ile His Ala Met
                   325                 330                 335
   Gly Pro Leu Leu Glu Gln Asn Gly Trp Pro Gly Ala His Phe Ile Thr
               340                 345                 350
   Asp Gln Gly Arg Ser Gly Lys Gln Pro Thr Gly Gln Ile Gln Trp Gly
           355                 360                 365
   Asp Trp Cys Asn Ser Lys Gly Thr Gly Phe Gly Ile Arg Pro Ser Ala
       370                 375                 380
   Asn Thr Gly Asp Ser Leu Leu Asp Ala Phe Val Trp Val Lys Pro Gly
   385                 390                 395                 400
   Gly Glu Ser Asp Gly Thr Ser Asp Thr Ser Ala Ala Arg Tyr Asp Tyr
                   405                 410                 415
   His Cys Gly Ile Asp Gly Ala Val Lys Pro Ala Pro Glu Ala Gly Thr
               420                 425                 430
   Trp Phe Gln Ala Tyr Phe Glu Gln Leu Leu Thr Asn Ala Asn Pro Ser
           435                 440                 445
   Phe Leu
       450
<210> 5
   <211> 1362
   <212> **DNA**
   <213> Artificial Sequence
<220>
   <223> Clone 95B7
<400> 5
   gtgcctctag aggagcggca agcttgctca agcgtctggg gccaatgtgg tggccagaat      60
   tggtcgggtc cgacttgctg tgcttccgga agcacatgcg tctactccaa cgactattac     120
   tcccagtgtc ttcccggcgc tgcaagctca agctcgtcca cgcgcgccgc gtcgacgact     180
   tctcgagtat cccccacaac atcccggtcg agctccgcga cgcctccacc tggttctact     240
   actaccagag tacctccagt cggatcggga accgctacgt attcaggcaa cccttttgtt     300
   ggggtcactc cttgggccaa tgcatattac gcctctgaag ttagcagcct cgctattcct     360
   agcttgactg gagccatggc cactgctgca gcagctgtcg caaaggttcc ctcttttatg     420
   tggctagata ctcttgacaa gacccctctc atggagcaaa ccttggccga catccgcacc     480
   gccaacaaga atggcggtaa ctatgccgga cagtttgtgg tgtatgactt gccggatcgc     540
   gattgcgctg cccttgcctc gaatggcgaa tactctattg ccgatggtgg cgtcgccaaa     600
   tataagaact atatcgacac cattcgtcaa attgtcgtgg aatattccga tatccggacc     660
   ctcctggtta ttgagcctga ctctcttgcc aaccaggtga ccaacatgga tgtcgccaag     720
   tgtgccaagg ctcatgatgc ctacatcagc ctgacgaact acgccgtcac agaactgaac     780
   cttccaaatg ttgcgatgta tttggacgct ggcaatgcag gatggcttgg ctggccggca     840
   aaccaaggcc cggccgctaa actatttgca agcatctaca aggatgcagg caagccggcc     900
   gctcttcgcg gattggcaac caatgtcgcc aactacaacg cctggagcct cagcagcgct     960
   ccaccttaca cgcaaggcgc ctctatctac gacgagaaga gcttcatcca cgctatggga    1020
   cctcttcttg agcagaatgg ctggcctggt gcccacttca tcactgatca aggtcgatcg    1080
   ggaaagcagc ctaccggaca gatccagtgg ggagactggt gcaattccaa gggcaccgga    1140
   tttggtattc gcccatccgc aaacactggg gactcgttgc tggatgcttt tgtctgggtc    1200
   aagccaggcg gcgagtctga cggcaccagc gacaccagtg cgacccgata cgactaccac    1260
   tgtggtgctt ctgccgcctt gcaaccggcg cctgaggctg gtacctggtt ccaagcctac    1320
   tttgagcagc ttctcaagaa cgcaaaccca tcgttcctgt aa                       1362
<210> 6
   <211> 453
   <212> PRT
   <213> Artificial Sequence
<220>
   <223> Clone 95B7
<400> 6
   Val Pro Leu Glu Glu Arg Gln Ala Cys Ser Ser Val Trp Gly Gln Cys
   1               5                   10                  15
   Gly Gly Gln Asn Trp Ser Gly Pro Thr Cys Cys Ala Ser Gly Ser Thr
               20                  25                  30
   Cys Val Tyr Ser Asn Asp Tyr Tyr Ser Gln Cys Leu Pro Gly Ala Ala
           35                  40                  45
   Ser Ser Ser Ser Ser Thr Arg Ala Ala Ser Thr Thr Ser Arg Val Ser
       50                  55                  60
   Pro Thr Thr Ser Arg Ser Ser Ser Ala Thr Pro Pro Pro Gly Ser Thr
   65                  70                  75                  80
   Thr Thr Arg Val Pro Pro Val Gly Ser Gly Thr Ala Thr Tyr Ser Gly
                   85                  90                  95
   Asn Pro Phe Val Gly Val Thr Pro Trp Ala Asn Ala Tyr Tyr Ala Ser
               100                 105                 110
   Glu Val Ser Ser Leu Ala Ile Pro Ser Leu Thr Gly Ala Met Ala Thr
           115                 120                 125
   Ala Ala Ala Ala Val Ala Lys Val Pro Ser Phe Met Trp Leu Asp Thr
       130                 135                 140
   Leu Asp Lys Thr Pro Leu Met Glu Gln Thr Leu Ala Asp Ile Arg Thr
   145                 150                 155                 160
   Ala Asn Lys Asn Gly Gly Asn Tyr Ala Gly Gln Phe Val Val Tyr Asp
                   165                 170                 175
   Leu Pro Asp Arg Asp Cys Ala Ala Leu Ala Ser Asn Gly Glu Tyr Ser
               180                 185                 190
   Ile Ala Asp Gly Gly Val Ala Lys Tyr Lys Asn Tyr Ile Asp Thr Ile
           195                 200                 205
   Arg Gln Ile Val Val Glu Tyr Ser Asp Ile Arg Thr Leu Leu Val Ile
       210                 215                 220
   Glu Pro Asp Ser Leu Ala Asn Gln Val Thr Asn Met Asp Val Ala Lys
   225                 230                 235                 240
   Cys Ala Lys Ala His Asp Ala Tyr Ile Ser Leu Thr Asn Tyr Ala Val
                   245                 250                 255
   Thr Glu Leu Asn Leu Pro Asn Val Ala Met Tyr Leu Asp Ala Gly Asn
               260                 265                 270
   Ala Gly Trp Leu Gly Trp Pro Ala Asn Gln Gly Pro Ala Ala Lys Leu
           275                 280                 285
   Phe Ala Ser Ile Tyr Lys Asp Ala Gly Lys Pro Ala Ala Leu Arg Gly
       290                 295                 300
   Leu Ala Thr Asn Val Ala Asn Tyr Asn Ala Trp Ser Leu Ser Ser Ala
   305                 310                 315                 320
   Pro Pro Tyr Thr Gln Gly Ala Ser Ile Tyr Asp Glu Lys Ser Phe Ile
                   325                 330                 335
   His Ala Met Gly Pro Leu Leu Glu Gln Asn Gly Trp Pro Gly Ala His
               340                 345                 350
   Phe Ile Thr Asp Gln Gly Arg Ser Gly Lys Gln Pro Thr Gly Gln Ile
           355                 360                 365
   Gln Trp Gly Asp Trp Cys Asn Ser Lys Gly Thr Gly Phe Gly Ile Arg
       370                 375                 380
   Pro Ser Ala Asn Thr Gly Asp Ser Leu Leu Asp Ala Phe Val Trp Val
   385                 390                 395                 400
   Lys Pro Gly Gly Glu Ser Asp Gly Thr Ser Asp Thr Ser Ala Thr Arg
                   405                 410                 415
   Tyr Asp Tyr His Cys Gly Ala Ser Ala Ala Leu Gln Pro Ala Pro Glu
               420                 425                 430
   Ala Gly Thr Trp Phe Gln Ala Tyr Phe Glu Gln Leu Leu Lys Asn Ala
           435                 440                 445
   Asn Pro Ser Phe Leu
       450
<210> 7
   <211> 1323
   <212> DNA
   <213> Artificial Sequence
<220>
   <223> Clone 100F11
<400> 7
   gctcctgttg aagagcgtca gtcttgcagc aacggagtct ggtctcaatg tggtggtcag      60
   aactggtccg gtactccttg ctgcaccagt ggaaacaagt gtgtcaaggt caacgacttc     120
   tactcccagt gccagcctgg atctgcagac ccttctccca catccaccat tgtctccgcg     180
   acgaccacca aggctactac tactggtagt ggaggctctg tcacctcgcc tcctcctgtt     240
   gccaccaaca accctttttc tggggtcgat ctgtgggcca ataactatta ccgctctgaa     300
   gttagcactc tcgctattcc taagttgagc ggagccatgg ccactgctgc agcaaaggtc     360
   gcagatgttc cctcttttca gtggatggat acttatgacc acatctcctt catggaggag     420
   tctttggccg acatccgcaa ggccaacaag gctggcggta actatgccgg acagtttgtg     480
   gtgtatgact tgccggatcg cgattgcgct gccgctgcct cgaatggcga atactctctt     540
   gacaaggatg gcaagaacaa atataaggcc tatatcgcca agattaaggg tattctccag     600
   gactattccg atacccggat cattctggtt attgagcctg actctcttgc caacatggtg     660
   accaacatga acgtcccaaa gtgtgccaat gctgcttcag cctacaagga gctcaccatt     720
   cacgccctca aggagctgaa ccttccaaat gtttccatgt atatcgacgc tggccatggt     780
   ggatggcttg gctggccggc aaaccttcct ccggccgctc agctatacgg tcagctctac     840
   aaggatgcag gcaagccgtc tcgccttcgc ggattggtca ccaatgtctc caactacaac     900
   gcctggaagc tatccagcaa gccagactac acggagagca accccaacta cgacgagcag     960
   aagtacatcc acgctctgtc tcctcttctt gagcaggagg gctggcccgg tgccaagttc    1020
   atcgtcgatc aaggtcgatc gggaaagcag cctaccggac agaaggcttg gggagactgg    1080
   tgcaatgctc ccggcaccgg atttggtatt cgcccatccg caaacactgg ggactcgttg    1140
   ctggatgctt ttgtctgggt caagccaggc ggcgagtctg acggcaccag cgacaccagt    1200
   gcgacccgat acgactacca ctgtggtgct tctgccgcct tgcaaccggc gcctgaggct    1260
   ggtacctggt tccaagccta ctttgagcag cttctcacaa acgcaaaccc atcgttcctg    1320
   taa                                                                  1323
<210> 8
   <211> 440
   <212> PRT
   <213> Artificial Sequence
<220>
   <223> Clone 100F11
<400> 8
   Ala Pro Val Glu Glu Arg Gln Ser Cys Ser Asn Gly Val Trp Ser Gln
   1               5                   10                  15
   Cys Gly Gly Gln Asn Trp Ser Gly Thr Pro Cys Cys Thr Ser Gly Asn
   20 25 30
   Lys Cys Val Lys Val Asn Asp Phe Tyr Ser Gln Cys Gln Pro Gly Ser
   35 40 45
   Ala Asp Pro Ser Pro Thr Ser Thr Ile Val Ser Ala Thr Thr Thr Lys
   50 55 60
   Ala Thr Thr Thr Gly Ser Gly Gly Ser Val Thr Ser Pro Pro Pro Val
   65 70 75 80
   Ala Thr Asn Asn Pro Phe Ser Gly Val Asp Leu Trp Ala Asn Asn Tyr
   85 90 95
   Tyr Arg Ser Glu Val Ser Thr Leu Ala Ile Pro Lys Leu Ser Gly Ala
   100 105 110
   Met Ala Thr Ala Ala Ala Lys Val Ala Asp Val Pro Ser Phe Gln Trp
   115 120 125
   Met Asp Thr Tyr Asp His Ile Ser Phe Met Glu Glu Ser Leu Ala Asp
   130 135 140
   Ile Arg Lys Ala Asn Lys Ala Gly Gly Asn Tyr Ala Gly Gln Phe Val
   145 150 155 160
   Val Tyr Asp Leu Pro Asp Arg Asp Cys Ala Ala Ala Ala Ser Asn Gly
   165 170 175
   Glu Tyr Ser Leu Asp Lys Asp Gly Lys Asn Lys Tyr Lys Ala Tyr Ile
   180 185 190
   Ala Lys Ile Lys Gly Ile Leu Gln Asp Tyr Ser Asp Thr Arg Ile Ile
   195 200 205
   Leu Val Ile Glu Pro Asp Ser Leu Ala Asn Met Val Thr Asn Met Asn
   210 215 220
   Val Pro Lys Cys Ala Asn Ala Ala Ser Ala Tyr Lys Glu Leu Thr Ile
   225 230 235 240
   His Ala Leu Lys Glu Leu Asn Leu Pro Asn Val Ser Met Tyr Ile Asp
   245 250 255
   Ala Gly His Gly Gly Trp Leu Gly Trp Pro Ala Asn Leu Pro Pro Ala
   260 265 270
   Ala Gln Leu Tyr Gly Gln Leu Tyr Lys Asp Ala Gly Lys Pro Ser Arg
           275                 280                 285
   Leu Arg Gly Leu Val Thr Asn Val Ser Asn Tyr Asn Ala Trp Lys Leu
       290                 295                 300
   Ser Ser Lys Pro Asp Tyr Thr Glu Ser Asn Pro Asn Tyr Asp Glu Gln
   305                 310                 315                 320
   Lys Tyr Ile His Ala Leu Ser Pro Leu Leu Glu Gln Glu Gly Trp Pro
                   325                 330                 335
   Gly Ala Lys Phe Ile Val Asp Gln Gly Arg Ser Gly Lys Gln Pro Thr
               340                 345                 350
   Gly Gln Lys Ala Trp Gly Asp Trp Cys Asn Ala Pro Gly Thr Gly Phe
           355                 360                 365
   Gly Ile Arg Pro Ser Ala Asn Thr Gly Asp Ser Leu Leu Asp Ala Phe
       370                 375                 380
   Val Trp Val Lys Pro Gly Gly Glu Ser Asp Gly Thr Ser Asp Thr Ser
   385                 390                 395                 400
   Ala Thr Arg Tyr Asp Tyr His Cys Gly Ala Ser Ala Ala Leu Gln Pro
                   405                 410                 415
   Ala Pro Glu Ala Gly Thr Trp Phe Gln Ala Tyr Phe Glu Gln Leu Leu
               420                 425                 430
   Thr Asn Ala Asn Pro Ser Phe Leu
           435                 440
<210> 9
   <211> 1323
   <212> DNA
   <213> Artificial Sequence
<220>
   <223> Clone 139F12
<400> 9
   gctcctgttg aagagcgtca gtcttgcagc aacggagtct ggtctcaatg tggtggtcag      60
   aactggtccg gtactccttg ctgcaccagt ggaaacaagt gtgtcaaggt caacgacttc     120
   tactcccagt gccagcctgg atctgcagac ccttctccca catccaccat tgtctccgcg     180
   acgaccacca aggctactac tactggtagt ggaggctctg tcacctcgcc tcctcctgtt     240
   gccaccaaca accctttttc tggggtcgat ctgtgggcca ataactatta ccgctctgaa     300
   gttagcactc tcgctattcc taagttgagc ggagccatgg ccactgctgc agcaaaggtc     360
   gcagatgttc cctcttttca gtggatggat acttatgacc acatctcctt catggaggag     420
   tctttggccg acatccgcaa ggccaacaag gctggcggta actatgccgg acagtttgtg     480
   gtgtatgact tgccggatcg cgattgcgct gccgctgcct cgaatggcga atactctctt     540
   gacaaggatg gcaagaacaa atataaggcc tatatcgcca agattaaggg tattctccag     600
   gactattccg atacccggat cattctggtt attgagcctg actctcttgc caacatggtg     660
   accaacatga acgtcccaaa gtgtgccaat gctgcttcag cctacaagga gctcaccatt     720
   cacgccctca aggagctgaa ccttccaaat gtttccatgt atatcgacgc tggccatggt     780
   ggatggcttg gctggccggc aaaccttcct ccggccgctc agctatacgg tcagctctac     840
   aaggatgcag gcaagccgtc tcgccttcgc ggattggtca ccaatgtctc caactacaac     900
   gcctggaagc tatccagcaa gccagactac acggagagca accccaacta cgacgagcag     960
   aagtacatcc acgctctgtc tcctcttctt gagcaggagg gctggcccgg tgccaagttc    1020
   atcgtcgatc aaggtcgatc gggaaagcag cctaccggac agaaggcttg gggagactgg    1080
   tgcaatgctc ccggcaccgg atttggtctc cgcccatccg caaacactgg ggacgccttg    1140
   gtcgatgctt ttgtctgggt caagccaggc ggcgagtctg acggcaccag cgacaccagt    1200
   gcggctcgat acgactacca ctgtggtgct tctgccgcct tgcaaccggc gcctgaggct    1260
   ggtacctggt tccaagccta ctttgagcag cttctcaaga acgcaaaccc atcgttcctg    1320
   taa                                                                  1323
<210> 10
   <211> 440
   <212> PRT
   <213> Artificial Sequence
<220>
   <223> Clone 139F12
<400> 10
   Ala Pro Val Glu Glu Arg Gln Ser Cys Ser Asn Gly Val Trp Ser Gln
   1               5                   10                  15
   Cys Gly Gly Gln Asn Trp Ser Gly Thr Pro Cys Cys Thr Ser Gly Asn
               20                  25                  30
   Lys Cys Val Lys Val Asn Asp Phe Tyr Ser Gln Cys Gln Pro Gly Ser
           35                  40                  45
   Ala Asp Pro Ser Pro Thr Ser Thr Ile Val Ser Ala Thr Thr Thr Lys
       50                  55                  60
   Ala Thr Thr Thr Gly Ser Gly Gly Ser Val Thr Ser Pro Pro Pro Val
   65 70 75 80
   Ala Thr Asn Asn Pro Phe Ser Gly Val Asp Leu Trp Ala Asn Asn Tyr
   85 90 95
   Tyr Arg Ser Glu Val Ser Thr Leu Ala Ile Pro Lys Leu Ser Gly Ala
   100 105 110
   Met Ala Thr Ala Ala Ala Lys Val Ala Asp Val Pro Ser Phe Gln Trp
   115 120 125
   Met Asp Thr Tyr Asp His Ile Ser Phe Met Glu Glu Ser Leu Ala Asp
   130 135 140
   Ile Arg Lys Ala Asn Lys Ala Gly Gly Asn Tyr Ala Gly Gln Phe Val
   145 150 155 160
   Val Tyr Asp Leu Pro Asp Arg Asp Cys Ala Ala Ala Ala Ser Asn Gly
   165 170 175
   Glu Tyr Ser Leu Asp Lys Asp Gly Lys Asn Lys Tyr Lys Ala Tyr Ile
   180 185 190
   Ala Lys Ile Lys Gly Ile Leu Gln Asp Tyr Ser Asp Thr Arg Ile Ile
   195 200 205
   Leu Val Ile Glu Pro Asp Ser Leu Ala Asn Met Val Thr Asn Met Asn
   210 215 220
   Val Pro Lys Cys Ala Asn Ala Ala Ser Ala Tyr Lys Glu Leu Thr Ile
   225 230 235 240
   His Ala Leu Lys Glu Leu Asn Leu Pro Asn Val Ser Met Tyr Ile Asp
   245 250 255
   Ala Gly His Gly Gly Trp Leu Gly Trp Pro Ala Asn Leu Pro Pro Ala
   260 265 270
   Ala Gln Leu Tyr Gly Gln Leu Tyr Lys Asp Ala Gly Lys Pro Ser Arg
   275 280 285
   Leu Arg Gly Leu Val Thr Asn Val Ser Asn Tyr Asn Ala Trp Lys Leu
   290 295 300
   Ser Ser Lys Pro Asp Tyr Thr Glu Ser Asn Pro Asn Tyr Asp Glu Gln
   305 310 315 320
   Lys Tyr Ile His Ala Leu Ser Pro Leu Leu Glu Gln Glu Gly Trp Pro
                   325                 330                 335
   Gly Ala Lys Phe Ile Val Asp Gln Gly Arg Ser Gly Lys Gln Pro Thr
               340                 345                 350
   Gly Gln Lys Ala Trp Gly Asp Trp Cys Asn Ala Pro Gly Thr Gly Phe
           355                 360                 365
   Gly Leu Arg Pro Ser Ala Asn Thr Gly Asp Ala Leu Val Asp Ala Phe
       370                 375                 380
   Val Trp Val Lys Pro Gly Gly Glu Ser Asp Gly Thr Ser Asp Thr Ser
   385                 390                 395                 400
   Ala Ala Arg Tyr Asp Tyr His Cys Gly Ala Ser Ala Ala Leu Gln Pro
                   405                 410                 415
   Ala Pro Glu Ala Gly Thr Trp Phe Gln Ala Tyr Phe Glu Gln Leu Leu
               420                 425                 430
   Lys Asn Ala Asn Pro Ser Phe Leu
           435                 440
<210> 11
   <211> 1323
   <212> DNA
   <213> Artificial Sequence
<220>
   <223> Clone 157B11
<400> 11
   gctcctgttg aagagcgtca gtcttgcagc aacggagtct ggtctcaatg tggtggtcag      60
   aactggtccg gtactccttg ctgcaccagt ggaaacaagt gtgtcaaggt caacgacttc     120
   tactcccagt gccagcctgg atctgcagac ccttctccca catccaccat tgtctccgcg     180
   acgaccacca aggctactac tactggtagt ggaggctctg tcacctcgcc tcctcctgtt     240
   gccaccaaca accctttttc tggggtcgat ctgtgggcca ataactatta ccgctctgaa     300
   gttagcactc tcgctattcc taagttgagc ggagccatgg ccactgctgc agcaaaggtc     360
   gcagatgttc cctcttttca gtggatggat acttatgacc acatctcctt catggaggag     420
   tctttggccg acatccgcaa ggccaacaag gctggcggta actatgccgg acagtttgtg     480
   gtgtatgact tgccggatcg cgattgcgct gccgccgcct cgaatggcga atactctctc     540
   gacaatgatg gcgccaacaa atataaggcc tatatcgcca agattaaggg tattctccag     600
   gactattccg atacccggat cattctggtt attgagcctg actctcttgc caacatggtg     660
   accaacatga acgtcccaaa gtgtgccaat gctgcttcag cctacaagga gctcaccatt     720
   cacgccctca aggagctgaa ccttccaaat gtttccatgt atatcgacgc tggccatggt     780
   ggatggcttg gctggccggc aaaccttcct ccggccgctc agctatacgg tcagctctac     840
   aaggatgcag gcaagccgtc tcgccttcgc ggattggtca ccaatgtctc caactacaac     900
   gcctggaagc tatccagcaa gccagactac acggagagca accccaacta cgacgagcag     960
   aagtacatcc acgctctgtc tcctcttctt gagcaggagg gctggcccgg tgccaagttc    1020
   atcgtcgatc aaggtcgatc gggaaagcag cctaccggac agaaggcttg gggagactgg    1080
   tgcaatgctc ccggcaccgg atttggtctc cgcccatccg caaacactgg ggactcgttg    1140
   ctggatgctt ttgtctgggt caagccaggc ggcgagtctg acggcaccag cgacaccagt    1200
   gcgacccgat acgactacca ctgtggtgct tctgccgcct tgcaaccggc gcctgaggct    1260
   ggtacctggt tccaagccta ctttgagcag cttctcacaa acgcaaaccc atcgttcctg    1320
   taa                                                                   1323
<210> 12
   <211> 440
   <212> PRT
   <213> Artificial Sequence
<220>
   <223> Clone 157B11
<400> 12
   Ala Pro Val Glu Glu Arg Gln Ser Cys Ser Asn Gly Val Trp Ser Gln
   1               5                   10                  15
   Cys Gly Gly Gln Asn Trp Ser Gly Thr Pro Cys Cys Thr Ser Gly Asn
               20                  25                  30
   Lys Cys Val Lys Val Asn Asp Phe Tyr Ser Gln Cys Gln Pro Gly Ser
           35                  40                  45
   Ala Asp Pro Ser Pro Thr Ser Thr Ile Val Ser Ala Thr Thr Thr Lys
       50                  55                  60
   Ala Thr Thr Thr Gly Ser Gly Gly Ser Val Thr Ser Pro Pro Pro Val
   65                  70                  75                  80
   Ala Thr Asn Asn Pro Phe Ser Gly Val Asp Leu Trp Ala Asn Asn Tyr
                   85                  90                  95
   Tyr Arg Ser Glu Val Ser Thr Leu Ala Ile Pro Lys Leu Ser Gly Ala
               100                 105                 110
   Met Ala Thr Ala Ala Ala Lys Val Ala Asp Val Pro Ser Phe Gln Trp
   115 120 125
   Met Asp Thr Tyr Asp His Ile Ser Phe Met Glu Glu Ser Leu Ala Asp
   130 135 140
   Ile Arg Lys Ala Asn Lys Ala Gly Gly Asn Tyr Ala Gly Gln Phe Val
   145 150 155 160
   Val Tyr Asp Leu Pro Asp Arg Asp Cys Ala Ala Ala Ala Ser Asn Gly
   165 170 175
   Glu Tyr Ser Leu Asp Asn Asp Gly Ala Asn Lys Tyr Lys Ala Tyr Ile
   180 185 190
   Ala Lys Ile Lys Gly Ile Leu Gln Asp Tyr Ser Asp Thr Arg Ile Ile
   195 200 205
   Leu Val Ile Glu Pro Asp Ser Leu Ala Asn Met Val Thr Asn Met Asn
   210 215 220
   Val Pro Lys Cys Ala Asn Ala Ala Ser Ala Tyr Lys Glu Leu Thr Ile
   225 230 235 240
   His Ala Leu Lys Glu Leu Asn Leu Pro Asn Val Ser Met Tyr Ile Asp
   245 250 255
   Ala Gly His Gly Gly Trp Leu Gly Trp Pro Ala Asn Leu Pro Pro Ala
   260 265 270
   Ala Gln Leu Tyr Gly Gln Leu Tyr Lys Asp Ala Gly Lys Pro Ser Arg
   275 280 285
   Leu Arg Gly Leu Val Thr Asn Val Ser Asn Tyr Asn Ala Trp Lys Leu
   290 295 300
   Ser Ser Lys Pro Asp Tyr Thr Glu Ser Asn Pro Asn Tyr Asp Glu Gln
   305 310 315 320
   Lys Tyr Ile His Ala Leu Ser Pro Leu Leu Glu Gln Glu Gly Trp Pro
   325 330 335
   Gly Ala Lys Phe Ile Val Asp Gln Gly Arg Ser Gly Lys Gln Pro Thr
   340 345 350
   Gly Gln Lys Ala Trp Gly Asp Trp Cys Asn Ala Pro Gly Thr Gly Phe
   355 360 365
   Gly Leu Arg Pro Ser Ala Asn Thr Gly Asp Ser Leu Leu Asp Ala Phe
   370 375 380
   Val Trp Val Lys Pro Gly Gly Glu Ser Asp Gly Thr Ser Asp Thr Ser
   385 390 395 400
   Ala Thr Arg Tyr Asp Tyr His Cys Gly Ala Ser Ala Ala Leu Gln Pro
   405 410 415
   Ala Pro Glu Ala Gly Thr Trp Phe Gln Ala Tyr Phe Glu Gln Leu Leu
   420 425 430
   Thr Asn Ala Asn Pro Ser Phe Leu
   435 440
<210> 13
   <211> 1323
   <212> DNA
   <213> Artificial Sequence
<220>
   <223> Clone 161A1
<400> 13
   gctcctgttg aagagcgtca gtcttgcagc aacggagtct ggtctcaatg tggtggtcag 60
   aactggtccg gtactccttg ctgcaccagt ggaaacaagt gtgtcaaggt caacgacttc 120
   tactcccagt gccagcctgg atctgcagac ccttctccca catccaccat tgtctccgcg 180
   acgaccacca aggctactac tactggtagt ggaggctctg tcacctcgcc tcctcctgtt 240
   gccaccaaca accctttttc tggggtcgat ctgtgggcca ataactatta ccgctctgaa 300
   gttagcactc tcgctattcc taagttgagc ggagccatgg ccactgctgc agcaaaggtc 360
   gcagatgttc cctcttttca gtggatggat acttatgacc acatctcctt catggaggag 420
   tctttggccg acatccgcaa ggccaacaag gctggcggta actatgccgg acagtttgtg 480
   gtgtatgact tgccggatcg cgattgcgct gccgctgcct cgaatggcga atactctctt 540
   gacaaggatg gcaagaacaa atataaggcc tatatcgcca agattaaggg tattctccag 600
   gactattccg atacccggat cattctggtt attgagcctg actctcttgc caacatggtg 660
   accaacatga acgtcccaaa gtgtgccaat gctgcttcag cctacaagga gctcaccatt 720
   cacgccctca aggagctgaa ccttccaaat gtttccatgt atatcgacgc tggccatggt 780
   ggatggcttg gctggccggc aaaccttcct ccggccgctc agctatacgg tcagctctac 840
   aaggatgcag gcaagccgtc tcgccttcgc ggattggtca ccaatgtctc caactacaac 900
   gcctggaagc tatccagcaa gccagactac acggagagca accccaacta cgacgagcag 960
   aagtacatcc acgctctgtc tcctcttctt gagcaggagg gctggcccgg tgccaagttc      1020
   atcgtcgatc aaggtcgatc gggaaagcag cctaccggac agaaggcttg gggagactgg      1080
   tgcaatgctc ccggcaccgg atttggtatc cgcccatccg caaacactgg ggacgccttg      1140
   gtcgatgctt ttgtctgggt caagccaggc ggcgagtctg acggcaccag cgacaccagt      1200
   gcgacccgat acgactacca ctgtggtgct tctgccgcct tgcaaccggc gcctgaggct      1260
   ggtacctggt tccaagccta ctttgagcag cttctcaaga acgcaaaccc atcgttcctg      1320
   taa                                                                    1323
<210> 14
   <211> 440
   <212> PRT
   <213> Artificial Sequence
<220>
   <223> Clone 161A1
<400> 14
   Ala Pro Val Glu Glu Arg Gln Ser Cys Ser Asn Gly Val Trp Ser Gln
   1               5                   10                  15
   Cys Gly Gly Gln Asn Trp Ser Gly Thr Pro Cys Cys Thr Ser Gly Asn
               20                  25                  30
   Lys Cys Val Lys Val Asn Asp Phe Tyr Ser Gln Cys Gln Pro Gly Ser
           35                  40                  45
   Ala Asp Pro Ser Pro Thr Ser Thr Ile Val Ser Ala Thr Thr Thr Lys
       50                  55                  60
   Ala Thr Thr Thr Gly Ser Gly Gly Ser Val Thr Ser Pro Pro Pro Val
   65                  70                  75                  80
   Ala Thr Asn Asn Pro Phe Ser Gly Val Asp Leu Trp Ala Asn Asn Tyr
                   85                  90                  95
   Tyr Arg Ser Glu Val Ser Thr Leu Ala Ile Pro Lys Leu Ser Gly Ala
               100                 105                 110
   Met Ala Thr Ala Ala Ala Lys Val Ala Asp Val Pro Ser Phe Gln Trp
           115                 120                 125
   Met Asp Thr Tyr Asp His Ile Ser Phe Met Glu Glu Ser Leu Ala Asp
       130                 135                 140
   Ile Arg Lys Ala Asn Lys Ala Gly Gly Asn Tyr Ala Gly Gln Phe Val
   145                 150                 155                 160
   Val Tyr Asp Leu Pro Asp Arg Asp Cys Ala Ala Ala Ala Ser Asn Gly
                   165                 170                 175
   Glu Tyr Ser Leu Asp Lys Asp Gly Lys Asn Lys Tyr Lys Ala Tyr Ile
               180                 185                 190
   Ala Lys Ile Lys Gly Ile Leu Gln Asp Tyr Ser Asp Thr Arg Ile Ile
           195                 200                 205
   Leu Val Ile Glu Pro Asp Ser Leu Ala Asn Met Val Thr Asn Met Asn
       210                 215                 220
   Val Pro Lys Cys Ala Asn Ala Ala Ser Ala Tyr Lys Glu Leu Thr Ile
   225                 230                 235                 240
   His Ala Leu Lys Glu Leu Asn Leu Pro Asn Val Ser Met Tyr Ile Asp
                   245                 250                 255
   Ala Gly His Gly Gly Trp Leu Gly Trp Pro Ala Asn Leu Pro Pro Ala
               260                 265                 270
   Ala Gln Leu Tyr Gly Gln Leu Tyr Lys Asp Ala Gly Lys Pro Ser Arg
           275                 280                 285
   Leu Arg Gly Leu Val Thr Asn Val Ser Asn Tyr Asn Ala Trp Lys Leu
       290                 295                 300
   Ser Ser Lys Pro Asp Tyr Thr Glu Ser Asn Pro Asn Tyr Asp Glu Gln
   305                 310                 315                 320
   Lys Tyr Ile His Ala Leu Ser Pro Leu Leu Glu Gln Glu Gly Trp Pro
                   325                 330                 335
   Gly Ala Lys Phe Ile Val Asp Gln Gly Arg Ser Gly Lys Gln Pro Thr
               340                 345                 350
   Gly Gln Lys Ala Trp Gly Asp Trp Cys Asn Ala Pro Gly Thr Gly Phe
           355                 360                 365
   Gly Ile Arg Pro Ser Ala Asn Thr Gly Asp Ala Leu Val Asp Ala Phe
       370                 375                 380
   Val Trp Val Lys Pro Gly Gly Glu Ser Asp Gly Thr Ser Asp Thr Ser
   385                 390                 395                 400
   Ala Thr Arg Tyr Asp Tyr His Cys Gly Ala Ser Ala Ala Leu Gln Pro
   405 410 415
   Ala Pro Glu Ala Gly Thr Trp Phe Gln Ala Tyr Phe Glu Gln Leu Leu
   420 425 430
   Lys Asn Ala Asn Pro Ser Phe Leu
   435 440
<210> 15
   <211> 1362
   <212> DNA
   <213> Artificial Sequence
<220>
   <223> Clone 161C12
<400> 15
   gtgcctctag aggagcggca agcttgctca agcgtctggg gccaatgtgg tggccagaat      60
   tggtcgggtc cgacttgctg tgcttccgga agcacatgcg tctactccaa cgactattac     120
   tcccagtgtc ttcccggcgc tgcaagctca agctcgtcca cgcgcgccgc gtcgacgact     180
   tctcgagtat cccccacaac atcccggtcg agctccgcga cgcctccacc tggttctact     240
   actaccagag tacctccagt cggatcggga accgctacgt attcaggcaa cccttttgtt    300
   ggggtcactc cttgggccaa tgcatattac gcctctgaag ttagcagcct cgctattcct     360
   agcttgactg gagccatggc cactgctgca gcagctgtcg caaaggttcc ctcttttatg     420
   tggctagata ctcttgacaa gacccctctc atggagcaaa ccttggccga catccgcacc     480
   gccaacaaga atggcggtaa ctatgccgga cagtttgtgg tgtatgactt gccggatcgc     540
   gattgcgctg cccttgcctc gaatggcgaa tactctctcg acaatgatgg cgccaacaaa     600
   tataagaact atatccaaac cattaagaag attatccaga gctattccga tatccggata     660
   ctcctggtta ttgagcctga ctctcttgcc aacctggtga ccaacatgga tgtcgccaag     720
   tgtgccaagg ctcatgatgc ctacatcagc ctgacgaact acgccgtcac agaactgaac     780
   cttccaaatg ttgcgatgta tttggacgct ggccatgcag gatggcttgg ctggccggca     840
   aaccttcctc cggccgctca gctatacggt cagctctaca aggatgcagg caagccgtct     900
   cgccttcgcg gattggtcac caatgtctcc aactacaacg cctggaagct atccagcaag     960
   ccagactaca cggagagcaa ccccaactac gacgagcaga agtacatcca cgctctgtct    1020
   cctcttcttg agcaggaggg ctggcccggt gccaagttca tcgtcgatca aggtcgatcg    1080
   ggaaagcagc ctaccggaca gaaggcttgg ggagactggt gcaatgctcc cggcaccgga    1140
   tttggtctcc gcccatccgc aaacactggg gacgccttgg tcgatgcttt tgtctgggtc    1200
   aagccaggcg gcgagtctga cggcaccagc gacaccagtg cggctcgata cgactaccac    1260
   tgtggtattg acggcgccgt caagccggcg cctgaggctg gtacctggtt ccaagcctac    1320
   tttgagcagc ttctcaagaa cgcaaaccca tcgttcctgt aa                       1362
<210> 16
   <211> 453
   <212> PRT
   <213> Artificial Sequence
<220>
   <223> Clone 161C12
<400> 16
   Val Pro Leu Glu Glu Arg Gln Ala Cys Ser Ser Val Trp Gly Gln Cys
   1               5                   10                  15
   Gly Gly Gln Asn Trp Ser Gly Pro Thr Cys Cys Ala Ser Gly Ser Thr
               20                  25                  30
   Cys Val Tyr Ser Asn Asp Tyr Tyr Ser Gln Cys Leu Pro Gly Ala Ala
           35                  40                  45
   Ser Ser Ser Ser Ser Thr Arg Ala Ala Ser Thr Thr Ser Arg Val Ser
       50                  55                  60
   Pro Thr Thr Ser Arg Ser Ser Ser Ala Thr Pro Pro Pro Gly Ser Thr
   65                  70                  75                  80
   Thr Thr Arg Val Pro Pro Val Gly Ser Gly Thr Ala Thr Tyr Ser Gly
                   85                  90                  95
   Asn Pro Phe Val Gly Val Thr Pro Trp Ala Asn Ala Tyr Tyr Ala Ser
               100                 105                 110
   Glu Val Ser Ser Leu Ala Ile Pro Ser Leu Thr Gly Ala Met Ala Thr
           115                 120                 125
   Ala Ala Ala Ala Val Ala Lys Val Pro Ser Phe Met Trp Leu Asp Thr
       130                 135                 140
   Leu Asp Lys Thr Pro Leu Met Glu Gln Thr Leu Ala Asp Ile Arg Thr
   145                 150                 155                 160
   Ala Asn Lys Asn Gly Gly Asn Tyr Ala Gly Gln Phe Val Val Tyr Asp
                   165                 170                 175
   Leu Pro Asp Arg Asp Cys Ala Ala Leu Ala Ser Asn Gly Glu Tyr Ser
               180                 185                 190
   Leu Asp Asn Asp Gly Ala Asn Lys Tyr Lys Asn Tyr Ile Gln Thr Ile
   195 200 205
   Lys Lys Ile Ile Gln Ser Tyr Ser Asp Ile Arg Ile Leu Leu Val Ile
   210 215 220
   Glu Pro Asp Ser Leu Ala Asn Leu Val Thr Asn Met Asp Val Ala Lys
   225 230 235 240
   Cys Ala Lys Ala His Asp Ala Tyr Ile Ser Leu Thr Asn Tyr Ala Val
   245 250 255
   Thr Glu Leu Asn Leu Pro Asn Val Ala Met Tyr Leu Asp Ala Gly His
   260 265 270
   Ala Gly Trp Leu Gly Trp Pro Ala Asn Leu Pro Pro Ala Ala Gln Leu
   275 280 285
   Tyr Gly Gln Leu Tyr Lys Asp Ala Gly Lys Pro Ser Arg Leu Arg Gly
   290 295 300
   Leu Val Thr Asn Val Ser Asn Tyr Asn Ala Trp Lys Leu Ser Ser Lys
   305 310 315 320
   Pro Asp Tyr Thr Glu Ser Asn Pro Asn Tyr Asp Glu Gln Lys Tyr Ile
   325 330 335
   His Ala Leu Ser Pro Leu Leu Glu Gln Glu Gly Trp Pro Gly Ala Lys
   340 345 350
   Phe Ile Val Asp Gln Gly Arg Ser Gly Lys Gln Pro Thr Gly Gln Lys
   355 360 365
   Ala Trp Gly Asp Trp Cys Asn Ala Pro Gly Thr Gly Phe Gly Leu Arg
   370 375 380
   Pro Ser Ala Asn Thr Gly Asp Ala Leu Val Asp Ala Phe Val Trp Val
   385 390 395 400
   Lys Pro Gly Gly Glu Ser Asp Gly Thr Ser Asp Thr Ser Ala Ala Arg
   405 410 415
   Tyr Asp Tyr His Cys Gly Ile Asp Gly Ala Val Lys Pro Ala Pro Glu
   420 425 430
   Ala Gly Thr Trp Phe Gln Ala Tyr Phe Glu Gln Leu Leu Lys Asn Ala
   435 440 445
   Asn Pro Ser Phe Leu
   450
<210> 17
   <211> 1323
   <212> DNA
   <213> Artificial Sequence
<220>
   <223> Clone 189H8
<400> 17
   gctcctgttg aagagcgtca gtcttgcagc aacggagtct ggtctcaatg tggtggtcag 60
   aactggtccg gtactccttg ctgcaccagt ggaaacaagt gtgtcaaggt caacgacttc 120
   tactcccagt gccagcctgg atctgcagac ccttctccca catccaccat tgtctccgcg 180
   acgaccacca aggctactac tactggtagt ggaggctctg tcacctcgcc tcctcctgtt 240
   gccaccaaca accctttttc tggggtcgat ctgtgggcca ataactatta ccgctctgaa 300
   gttagcactc tcgctattcc taagttgagc ggagccatgg ccactgctgc agcaaaggtc 360
   gcagatgttc cctcttttca gtggatggat acttatgacc acatctccct catggaggac 420
   accttggtcg acatccgcaa ggccaacctg gctggcggta actatgccgg acagtttgtg 480
   gtgtatgact tgccggatcg cgattgcgct gccgctgcct cgaatggcga atactctctt 540
   gacaaggatg gcaagaacaa atataaggcc tatatcgcca agattaaggg tattctccag 600
   gactattccg atacccggat cattctggtt attgagcctg actctcttgc caacatggtg 660
   accaacatga acgtcccaaa gtgtgccaat gctgcttcag cctacaagga gctcaccatt 720
   cacgccctca aggagctgaa ccttccaaat gtttccatgt atatcgacgc tggccatggt 780
   ggatggcttg gctggccggc aaaccaaggc ccggccgcta aactatttgc aagcatctac 840
   aaggatgcag gcaagccggc cgctcttcgc ggattggcaa ccaatgtcgc caactacaac 900
   gcctggagcc tcagcagcgc tccaccttac acgcaaggcg cctctatcta cgacgagaag 960
   agcttcatcc acgctatggg acctcttctt gagcagaatg gctggcctgg tgccaagttc 1020
   atcgtcgatc aaggtcgatc gggaaagcag cctaccggac agaaggcttg gggagactgg 1080
   tgcaatgctc ccggcaccgg atttggtctc cgcccatccg caaacactgg ggactcgttg 1140
   ctggatgctt ttgtctgggt caagccaggc ggcgagtctg acggcaccag cgacaccagt 1200
   gcgacccgat acgactacca ctgtggtgct tctgccgcct tgcaaccggc gcctgaggct 1260
   ggtacctggt tccaagccta ctttgagcag cttctcacaa acgcaaaccc atcgttcctg 1320
   taa 1323
<210> 18
   <211> 440
   <212> PRT
   <213> Artificial Sequence
<220>
   <223> Clone 189H8
<400> 18
   Ala Pro Val Glu Glu Arg Gln Ser Cys Ser Asn Gly Val Trp Ser Gln
   1               5                   10                  15
   Cys Gly Gly Gln Asn Trp Ser Gly Thr Pro Cys Cys Thr Ser Gly Asn
               20                  25                  30
   Lys Cys Val Lys Val Asn Asp Phe Tyr Ser Gln Cys Gln Pro Gly Ser
           35                  40                  45
   Ala Asp Pro Ser Pro Thr Ser Thr Ile Val Ser Ala Thr Thr Thr Lys
       50                  55                  60
   Ala Thr Thr Thr Gly Ser Gly Gly Ser Val Thr Ser Pro Pro Pro Val
   65                  70                  75                  80
   Ala Thr Asn Asn Pro Phe Ser Gly Val Asp Leu Trp Ala Asn Asn Tyr
                   85                  90                  95
   Tyr Arg Ser Glu Val Ser Thr Leu Ala Ile Pro Lys Leu Ser Gly Ala
               100                 105                 110
   Met Ala Thr Ala Ala Ala Lys Val Ala Asp Val Pro Ser Phe Gln Trp
           115                 120                 125
   Met Asp Thr Tyr Asp His Ile Ser Leu Met Glu Asp Thr Leu Val Asp
       130                 135                 140
   Ile Arg Lys Ala Asn Leu Ala Gly Gly Asn Tyr Ala Gly Gln Phe Val
   145                 150                 155                 160
   Val Tyr Asp Leu Pro Asp Arg Asp Cys Ala Ala Ala Ala Ser Asn Gly
                   165                 170                 175
   Glu Tyr Ser Leu Asp Lys Asp Gly Lys Asn Lys Tyr Lys Ala Tyr Ile
               180                 185                 190
   Ala Lys Ile Lys Gly Ile Leu Gln Asp Tyr Ser Asp Thr Arg Ile Ile
           195                 200                 205
   Leu Val Ile Glu Pro Asp Ser Leu Ala Asn Met Val Thr Asn Met Asn
       210                 215                 220
   Val Pro Lys Cys Ala Asn Ala Ala Ser Ala Tyr Lys Glu Leu Thr Ile
   225                 230                 235                 240
   His Ala Leu Lys Glu Leu Asn Leu Pro Asn Val Ser Met Tyr Ile Asp
                   245                 250                 255
   Ala Gly His Gly Gly Trp Leu Gly Trp Pro Ala Asn Gln Gly Pro Ala
               260                 265                 270
   Ala Lys Leu Phe Ala Ser Ile Tyr Lys Asp Ala Gly Lys Pro Ala Ala
           275                 280                 285
   Leu Arg Gly Leu Ala Thr Asn Val Ala Asn Tyr Asn Ala Trp Ser Leu
       290                 295                 300
   Ser Ser Ala Pro Pro Tyr Thr Gln Gly Ala Ser Ile Tyr Asp Glu Lys
   305                 310                 315                 320
   Ser Phe Ile His Ala Met Gly Pro Leu Leu Glu Gln Asn Gly Trp Pro
                   325                 330                 335
   Gly Ala Lys Phe Ile Val Asp Gln Gly Arg Ser Gly Lys Gln Pro Thr
               340                 345                 350
   Gly Gln Lys Ala Trp Gly Asp Trp Cys Asn Ala Pro Gly Thr Gly Phe
           355                 360                 365
   Gly Leu Arg Pro Ser Ala Asn Thr Gly Asp Ser Leu Leu Asp Ala Phe
       370                 375                 380
   Val Trp Val Lys Pro Gly Gly Glu Ser Asp Gly Thr Ser Asp Thr Ser
   385                 390                 395                 400
   Ala Thr Arg Tyr Asp Tyr His Cys Gly Ala Ser Ala Ala Leu Gln Pro
                   405                 410                 415
   Ala Pro Glu Ala Gly Thr Trp Phe Gln Ala Tyr Phe Glu Gln Leu Leu
               420                 425                 430
   Thr Asn Ala Asn Pro Ser Phe Leu
           435                 440
<210> 19
   <211> 1362
   <212> DNA
   <213> Artificial Sequence
<220>
   <223> Clone 196D9
<400> 19
   gtgcctctag aggagcggca agcttgctca agcgtctggg gccaatgtgg tggccagaat      60
   tggtcgggtc cgacttgctg tgcttccgga agcacatgcg tctactccaa cgactattac     120
   tcccagtgtc ttcccggcgc tgcaagctca agctcgtcca cgcgcgccgc gtcgacgact     180
   tctcgagtat cccccacaac atcccggtcg agctccgcga cgcctccacc tggttctact     240
   actaccagag tacctccagt cggatcggga accgctacgt attcaggcaa cccttttgtt     300
   ggggtcactc cttgggccaa tgcatattac gcctctgaag ttagcagcct cgctattcct     360
   agcttgactg gagccatggc cactgctgca gcagctgtcg caaaggttcc ctcttttatg     420
   tggctagata ctcttgacaa gacccctctc atggagcaaa ccttggccga catccgcacc     480
   gccaacaaga atggcggtaa ctatgccgga cagtttgtgg tgtatgactt gccggatcgc     540
   gattgcgctg cccttgcctc gaatggcgaa tactctattg ccgatggtgg cgtcgccaaa     600
   tataagaact atatcgacac cattcgtcaa attgtcgtgg aatattccga tatccggacc     660
   ctcctggtta ttgagcctga ctctcttacc aacctggtga ccaacatgaa cgtcccaaag     720
   tgtgccaatg ctcagtcagc ctaccttgag tgcatcaact acgccgtcac acagctgaac     780
   cttccaaatg ttgcgatgta tttggacgct ggccatgcag gatggcttgg ctggccggca     840
   aaccaagacc cggccgctca gctatttgca aatgtttaca agaatgcatc gtctccgaga     900
   gctcttcgcg gattggcaac caatgtcgcc aactacaacg ggtggaacat taccagcccc     960
   ccatcgtaca cgcaaggcaa cgctgtctac aacgagaagc tgtacatcca cgctattgga    1020
   cctcttcttg ccaatcacgg ctggtccaac gccttcttca tcactgatca aggtcgatcg    1080
   ggaaagcagc ctaccggaca gcaacagtgg ggagactggt gcaatgctcc cggcaccgga    1140
   tttggtctcc gcccatccgc aaacactggg gacgccttgg tcgatgcttt tgtctgggtc    1200
   aagccaggcg gcgagtctga cggcaccagc gacaccagtg cggctcgata cgactaccac    1260
   tgtggtattg acggcgccgt caagccggcg cctgaggctg gtacctggtt ccaagcctac    1320
   tttgagcagc ttctcaagaa cgcaaaccca tcgttcctgt aa                       1362
<210> 20
   <211> 453
   <212> PRT
   <213> Artificial Sequence
<220>
   <223> Clone 196D9
<400> 20
   Val Pro Leu Glu Glu Arg Gln Ala Cys Ser Ser Val Trp Gly Gln Cys
   1               5                   10                  15
   Gly Gly Gln Asn Trp Ser Gly Pro Thr Cys Cys Ala Ser Gly Ser Thr
               20                  25                  30
   Cys Val Tyr Ser Asn Asp Tyr Tyr Ser Gln Cys Leu Pro Gly Ala Ala
           35                  40                  45
   Ser Ser Ser Ser Ser Thr Arg Ala Ala Ser Thr Thr Ser Arg Val Ser
       50                  55                  60
   Pro Thr Thr Ser Arg Ser Ser Ser Ala Thr Pro Pro Pro Gly Ser Thr
   65                  70                  75                  80
   Thr Thr Arg Val Pro Pro Val Gly Ser Gly Thr Ala Thr Tyr Ser Gly
                   85                  90                  95
   Asn Pro Phe Val Gly Val Thr Pro Trp Ala Asn Ala Tyr Tyr Ala Ser
               100                 105                 110
   Glu Val Ser Ser Leu Ala Ile Pro Ser Leu Thr Gly Ala Met Ala Thr
           115                 120                 125
   Ala Ala Ala Ala Val Ala Lys Val Pro Ser Phe Met Trp Leu Asp Thr
       130                 135                 140
   Leu Asp Lys Thr Pro Leu Met Glu Gln Thr Leu Ala Asp Ile Arg Thr
   145                 150                 155                 160
   Ala Asn Lys Asn Gly Gly Asn Tyr Ala Gly Gln Phe Val Val Tyr Asp
                   165                 170                 175
   Leu Pro Asp Arg Asp Cys Ala Ala Leu Ala Ser Asn Gly Glu Tyr Ser
               180                 185                 190
   Ile Ala Asp Gly Gly Val Ala Lys Tyr Lys Asn Tyr Ile Asp Thr Ile
           195                 200                 205
   Arg Gln Ile Val Val Glu Tyr Ser Asp Ile Arg Thr Leu Leu Val Ile
       210                 215                 220
   Glu Pro Asp Ser Leu Thr Asn Leu Val Thr Asn Met Asn Val Pro Lys
   225                 230                 235                 240
   Cys Ala Asn Ala Gln Ser Ala Tyr Leu Glu Cys Ile Asn Tyr Ala Val
                   245                 250                 255
   Thr Gln Leu Asn Leu Pro Asn Val Ala Met Tyr Leu Asp Ala Gly His
               260                 265                 270
   Ala Gly Trp Leu Gly Trp Pro Ala Asn Gln Asp Pro Ala Ala Gln Leu
           275                 280                 285
   Phe Ala Asn Val Tyr Lys Asn Ala Ser Ser Pro Arg Ala Leu Arg Gly
       290                 295                 300
   Leu Ala Thr Asn Val Ala Asn Tyr Asn Gly Trp Asn Ile Thr Ser Pro
   305                 310                 315                 320
   Pro Ser Tyr Thr Gln Gly Asn Ala Val Tyr Asn Glu Lys Leu Tyr Ile
                   325                 330                 335
   His Ala Ile Gly Pro Leu Leu Ala Asn His Gly Trp Ser Asn Ala Phe
               340                 345                 350
   Phe Ile Thr Asp Gln Gly Arg Ser Gly Lys Gln Pro Thr Gly Gln Gln
           355                 360                 365
   Gln Trp Gly Asp Trp Cys Asn Ala Pro Gly Thr Gly Phe Gly Leu Arg
       370                 375                 380
   Pro Ser Ala Asn Thr Gly Asp Ala Leu Val Asp Ala Phe Val Trp Val
   385                 390                 395                 400
   Lys Pro Gly Gly Glu Ser Asp Gly Thr Ser Asp Thr Ser Ala Ala Arg
                   405                 410                 415
   Tyr Asp Tyr His Cys Gly Ile Asp Gly Ala Val Lys Pro Ala Pro Glu
               420                 425                 430
   Ala Gly Thr Trp Phe Gln Ala Tyr Phe Glu Gln Leu Leu Lys Asn Ala
           435                 440                 445
   Asn Pro Ser Phe Leu
       450
<210> 21
   <211> 1362
   <212> DNA
   <213> Artificial Sequence
<220>
   <223> Clone 198E11
<400> 21
   gtgcctctag aggagcggca agcttgctca agcgtctggg gccaatgtgg tggccagaat      60
   tggtcgggtc cgacttgctg tgcttccgga agcacatgcg tctactccaa cgactattac     120
   tcccagtgtc ttcccggcgc tgcaagctca agctcgtcca cgcgcgccgc gtcgacgact     180
   tctcgagtat cccccacaac atcccggtcg agctccgcga cgcctccacc tggttctact     240
   actaccagag tacctccagt cggatcggga accgctacgt attcaggcaa cccttttgtt    300
   ggggtcactc cttgggccaa tgcatattac gcctctgaag ttagcagcct cgctattcct     360
   agcttgactg gagccatggc cactgctgca gcagctgtcg caaaggttcc ctcttttatg     420
   tggctagata ctcttgacaa gacccctctc atggagcaaa ccttggccga catccgcacc     480
   gccaacaaga atggcggtaa ctatgccgga cagtttgtgg tgtatgactt gccggatcgc     540
   gattgcgctg ccgctgcctc gaatggcgaa tactctcttg acaaggatgg caagaacaaa     600
   tataaggcct atatcgccaa gattaagggt attctccagg actattccga tacccggatc     660
   attctggtta ttgagcctga ctctcttgcc aacatggtga ccaacatgaa cgtcccaaag     720
   tgtgccaatg ctgcttcagc ctacaaggag ctcaccattc acgccctcaa ggagctgaac     780
   cttccaaatg tttccatgta tatcgacgct ggccatggtg gatggcttgg ctggccggca     840
   aaccttcctc cggccgctca gctatacggt cagctctaca aggatgcagg caagccgtct     900
   cgccttcgcg gattggtcac caatgtctcc aactacaacg cctggaagct atccagcaag     960
   ccagactaca cggagagcaa ccccaactac gacgagcaga agtacatcca cgctctgtct    1020
   cctcttcttg agcaggaggg ctggcccggt gccaagttca tcgtcgatca aggtcgatcg    1080
   ggaaagcagc ctaccggaca gaaggcttgg ggagactggt gcaatgtgat cggcaccgga    1140
   tttggtattc gcccatccgc aaacactggg gactcgttgc tggattcgtt tgtctgggtc    1200
   aagccaggcg gcgagtctga cggcaccagc gacaccagtg cgacccgata cgactaccac    1260
   tgtggtgctt ctgccgcctt gcaaccggcg cctgaggctg gtacctggtt ccaagcctac    1320
   tttgagcagc ttctcacaaa cgcaaaccca tcgttcctgt aa                        1362
<210> 22
   <211> 453
   <212> PRT
   <213> Artificial Sequence
<220>
   <223> Clone 198E11
<400> 22
   Val Pro Leu Glu Glu Arg Gln Ala Cys Ser Ser Val Trp Gly Gln Cys
   1               5                   10                  15
   Gly Gly Gln Asn Trp Ser Gly Pro Thr Cys Cys Ala Ser Gly Ser Thr
               20                  25                  30
   Cys Val Tyr Ser Asn Asp Tyr Tyr Ser Gln Cys Leu Pro Gly Ala Ala
           35                  40                  45
   Ser Ser Ser Ser Ser Thr Arg Ala Ala Ser Thr Thr Ser Arg Val Ser
       50                  55                  60
   Pro Thr Thr Ser Arg Ser Ser Ser Ala Thr Pro Pro Pro Gly Ser Thr
   65                  70                  75                  80
   Thr Thr Arg Val Pro Pro Val Gly Ser Gly Thr Ala Thr Tyr Ser Gly
                   85                  90                  95
   Asn Pro Phe Val Gly Val Thr Pro Trp Ala Asn Ala Tyr Tyr Ala Ser
               100                 105                 110
   Glu Val Ser Ser Leu Ala Ile Pro Ser Leu Thr Gly Ala Met Ala Thr
           115                 120                 125
   Ala Ala Ala Ala Val Ala Lys Val Pro Ser Phe Met Trp Leu Asp Thr
       130                 135                 140
   Leu Asp Lys Thr Pro Leu Met Glu Gln Thr Leu Ala Asp Ile Arg Thr
   145                 150                 155                 160
   Ala Asn Lys Asn Gly Gly Asn Tyr Ala Gly Gln Phe Val Val Tyr Asp
                   165                 170                 175
   Leu Pro Asp Arg Asp Cys Ala Ala Ala Ala Ser Asn Gly Glu Tyr Ser
               180                 185                 190
   Leu Asp Lys Asp Gly Lys Asn Lys Tyr Lys Ala Tyr Ile Ala Lys Ile
           195                 200                 205
   Lys Gly Ile Leu Gln Asp Tyr Ser Asp Thr Arg Ile Ile Leu Val Ile
       210                 215                 220
   Glu Pro Asp Ser Leu Ala Asn Met Val Thr Asn Met Asn Val Pro Lys
   225                 230                 235                 240
   Cys Ala Asn Ala Ala Ser Ala Tyr Lys Glu Leu Thr Ile His Ala Leu
                   245                 250                 255
   Lys Glu Leu Asn Leu Pro Asn Val Ser Met Tyr Ile Asp Ala Gly His
               260                 265                 270
   Gly Gly Trp Leu Gly Trp Pro Ala Asn Leu Pro Pro Ala Ala Gln Leu
           275                 280                 285
   Tyr Gly Gln Leu Tyr Lys Asp Ala Gly Lys Pro Ser Arg Leu Arg Gly
       290                 295                 300
   Leu Val Thr Asn Val Ser Asn Tyr Asn Ala Trp Lys Leu Ser Ser Lys
   305                 310                 315                 320
   Pro Asp Tyr Thr Glu Ser Asn Pro Asn Tyr Asp Glu Gln Lys Tyr Ile
                   325                 330                 335
   His Ala Leu Ser Pro Leu Leu Glu Gln Glu Gly Trp Pro Gly Ala Lys
               340                 345                 350
   Phe Ile Val Asp Gln Gly Arg Ser Gly Lys Gln Pro Thr Gly Gln Lys
           355                 360                 365
   Ala Trp Gly Asp Trp Cys Asn Val Ile Gly Thr Gly Phe Gly Ile Arg
       370                 375                 380
   Pro Ser Ala Asn Thr Gly Asp Ser Leu Leu Asp Ser Phe Val Trp Val
   385                 390                 395                 400
   Lys Pro Gly Gly Glu Ser Asp Gly Thr Ser Asp Thr Ser Ala Thr Arg
                   405                 410                 415
   Tyr Asp Tyr His Cys Gly Ala Ser Ala Ala Leu Gln Pro Ala Pro Glu
               420                 425                 430
   Ala Gly Thr Trp Phe Gln Ala Tyr Phe Glu Gln Leu Leu Thr Asn Ala
           435                 440                 445
   Asn Pro Ser Phe Leu
       450
<210> 23
   <211> 1323
   <212> DNA
   <213> Artificial Sequence
<220>
   <223> Clone 251B4
<400> 23
   gctcctgttg aagagcgtca gtcttgcagc aacggagtct ggtctcaatg tggtggtcag      60
   aactggtccg gtactccttg ctgcaccagt ggaaacaagt gtgtcaaggt caacgacttc     120
   tactcccagt gccagcctgg atctgcagac ccttctccca catccaccat tgtctccgcg     180
   acgaccacca aggctactac tactggtagt ggaggctctg tcacctcgcc tcctcctgtt     240
   gccaccaaca accctttttc tggggtcgat ctgtgggcca ataactatta ccgctctgaa      300
   gttagcactc tcgctattcc taagttgagc ggagccatgg ccactgctgc agcaaaggtc     360
   gcagatgttc cctcttttca gtggatggat acttatgacc acatctcctt catggaggag 420
   tctttggccg acatccgcac cgccaacaag aatggcggta actatgccgg acagtttgtg 480
   gtgtatgact tgccggatcg cgattgcgct gccgccgcct cgaatggcga atactctctc 540
   gacaatgatg gcgccaacaa atataagaac tatatccaaa ccattaagaa gattatccag 600
   agctattccg atatccggat actcctggtt attgagcctg actctcttgc caacctggtg 660
   accaacatgg atgtcgccaa gtgtgccaag gctcatgatg cctacatcag cctgacgaac 720
   tacgccgtca cagaactgaa ccttccaaat gttgcgatgt atttggacgc tggccatgca 780
   ggatggcttg gctggccggc aaaccaaggc ccggccgcta aactatttgc aagcatctac 840
   aaggatgcag gcaagccggc cgctcttcgc ggattggcaa ccaatgtcgc caactacaac 900
   gcctggaagc tatccagcaa gccagactac acggagagca accccaacta cgacgagcag 960
   aagtacatcc acgctctgtc tcctcttctt gagcaggagg gctggcccgg tgccaagttc 1020
   atcgtcgatc aaggtcgatc gggaaagcag cctaccggac agaaggcttg gggagactgg 1080
   tgcaatgctc ccggcaccgg atttggtctc cgcccatccg caaacactgg ggacgccttg 1140
   gtcgatgctt ttgtctgggt caagccaggc ggcgagtgtg acggcaccag cgacaccagt 1200
   gcggctcgat acgactacca ctgtggtatt gacggcgccg tcaagccggc gcctgaggct 1260
   ggtacctggt tccaagccta ctttgagcag cttctcacaa acgcaaaccc atcgttcctg 1320
   taa 1323
<210> 24
   <211> 440
   <212> PRT
   <213> Artificial Sequence
<220>
   <223> Clone 251B4
<400> 24
   Ala Pro Val Glu Glu Arg Gln Ser Cys Ser Asn Gly Val Trp Ser Gln
   1               5                   10                  15
   Cys Gly Gly Gln Asn Trp Ser Gly Thr Pro Cys Cys Thr Ser Gly Asn
               20                  25                  30
   Lys Cys Val Lys Val Asn Asp Phe Tyr Ser Gln Cys Gln Pro Gly Ser
           35                  40                  45
   Ala Asp Pro Ser Pro Thr Ser Thr Ile Val Ser Ala Thr Thr Thr Lys
       50                  55                  60
   Ala Thr Thr Thr Gly Ser Gly Gly Ser Val Thr Ser Pro Pro Pro Val
   65                  70                  75                  80
   Ala Thr Asn Asn Pro Phe Ser Gly Val Asp Leu Trp Ala Asn Asn Tyr
   85 90 95
   Tyr Arg Ser Glu Val Ser Thr Leu Ala Ile Pro Lys Leu Ser Gly Ala
   100 105 110
   Met Ala Thr Ala Ala Ala Lys Val Ala Asp Val Pro Ser Phe Gln Trp
   115 120 125
   Met Asp Thr Tyr Asp His Ile Ser Phe Met Glu Glu Ser Leu Ala Asp
   130 135 140
   Ile Arg Thr Ala Asn Lys Asn Gly Gly Asn Tyr Ala Gly Gln Phe Val
   145 150 155 160
   Val Tyr Asp Leu Pro Asp Arg Asp Cys Ala Ala Ala Ala Ser Asn Gly
   165 170 175
   Glu Tyr Ser Leu Asp Asn Asp Gly Ala Asn Lys Tyr Lys Asn Tyr Ile
   180 185 190
   Gln Thr Ile Lys Lys Ile Ile Gln Ser Tyr Ser Asp Ile Arg Ile Leu
   195 200 205
   Leu Val Ile Glu Pro Asp Ser Leu Ala Asn Leu Val Thr Asn Met Asp
   210 215 220
   Val Ala Lys Cys Ala Lys Ala His Asp Ala Tyr Ile Ser Leu Thr Asn
   225 230 235 240
   Tyr Ala Val Thr Glu Leu Asn Leu Pro Asn Val Ala Met Tyr Leu Asp
   245 250 255
   Ala Gly His Ala Gly Trp Leu Gly Trp Pro Ala Asn Gln Gly Pro Ala
   260 265 270
   Ala Lys Leu Phe Ala Ser Ile Tyr Lys Asp Ala Gly Lys Pro Ala Ala
   275 280 285
   Leu Arg Gly Leu Ala Thr Asn Val Ala Asn Tyr Asn Ala Trp Lys Leu
   290 295 300
   Ser Ser Lys Pro Asp Tyr Thr Glu Ser Asn Pro Asn Tyr Asp Glu Gln
   305 310 315 320
   Lys Tyr Ile His Ala Leu Ser Pro Leu Leu Glu Gln Glu Gly Trp Pro
   325 330 335
   Gly Ala Lys Phe Ile Val Asp Gln Gly Arg Ser Gly Lys Gln Pro Thr
   340 345 350
   Gly Gln Lys Ala Trp Gly Asp Trp Cys Asn Ala Pro Gly Thr Gly Phe
   355 360 365
   Gly Leu Arg Pro Ser Ala Asn Thr Gly Asp Ala Leu Val Asp Ala Phe
   370 375 380
   Val Trp Val Lys Pro Gly Gly Glu Cys Asp Gly Thr Ser Asp Thr Ser
   385 390 395 400
   Ala Ala Arg Tyr Asp Tyr His Cys Gly Ile Asp Gly Ala Val Lys Pro
   405 410 415
   Ala Pro Glu Ala Gly Thr Trp Phe Gln Ala Tyr Phe Glu Gln Leu Leu
   420 425 430
   Thr Asn Ala Asn Pro Ser Phe Leu
   435 440
<210> 25
   <211> 1362
   <212> DNA
   <213> Artificial Sequence
<220>
   <223> Clone 251C4
<400> 25
   gtgcctctag aggagcggca agcttgctca agcgtctggg gccaatgtgg tggccagaat      60
   tggtcgggtc cgacttgctg tgcttccgga agcacatgcg tctactccaa cgactattac     120
   tcccagtgtc ttcccggcgc tgcaagctca agctcgtcca cgcgcgccgc gtcgacgact     180
   tctcgagtat cccccacaac atcccggtcg agctccgcga cgcctccacc tggttctact     240
   actaccagag tacctccagt cggatcggga accgctacgt attcaggcaa cccttttgtt    300
   ggggtcactc cttgggccaa tgcatattac gcctctgaag ttagcagcct cgctattcct     360
   agcttgactg gagccatggc cactgctgca gcagctgtcg caaaggttcc ctcttttatg     420
   tggctagata ctcttgacaa gacccctctc atggagcaaa ccttggccga catccgcacc     480
   gccaacaaga atggcggtaa ctatgccgga cagtttgtgg tgtatgactt gccggatcgc     540
   gattgcgctg ccgctgcctc gaatggcgaa tactctattg ccgatggtgg cgtcgccaaa     600
   tataagaact atatcgacac cattcgtcaa attgtcgtgg aatattccga tatccggacc     660
   ctcctggtta ttgagcctga ctctcttgcc aacctggtga ccaacctcgg tactccaaag     720
   tgtgccaatg ctcagtcagc ctaccttgag tgcatcaact acgccgtcac acagctgaac     780
   cttccaaatg ttgcgatgta tttggacgct ggccatgcag gatggcttgg ctggccggca     840
   aaccaagacc cggccgctca gctatttgca aatgtttaca agaatgcatc gtctccgaga     900
   gctcttcgcg gattggcaac caatgtcgcc aactacaacg ggtggaacat taccagcccc     960
   ccatcgtaca cgcaaggcaa cgctgtctac aacgagaagc tgtacatcca cgctattgga    1020
   cctcttcttg ccaatcacgg ctggtccaac gccttcttca tcactgatca aggtcgatcg    1080
   ggaaagcagc ctaccggaca gcaacagtgg ggagactggt gcaatgtgat cggcaccgga    1140
   tttggtattc gcccatccgc aaacactggg gactcgttgc tggattcgtt tgtctgggtc    1200
   aagccaggcg gcgagtgtga cggcaccagc gacaccagtg cgccacgatt tgactcccac    1260
   tgtgcgctcc cagatgcctt gcaaccggcg cctgaggctg gtacctggtt ccaagcctac    1320
   tttgagcagc ttctcacaaa cgcaaaccca tcgttcctgt aa                        1362
<210> 26
   <211> 453
   <212> PRT
   <213> Artificial Sequence
<220>
   <223> Clone 251C4
<400> 26
   Val Pro Leu Glu Glu Arg Gln Ala Cys Ser Ser Val Trp Gly Gln Cys
   1               5                   10                  15
   Gly Gly Gln Asn Trp Ser Gly Pro Thr Cys Cys Ala Ser Gly Ser Thr
               20                  25                  30
   Cys Val Tyr Ser Asn Asp Tyr Tyr Ser Gln Cys Leu Pro Gly Ala Ala
           35                  40                  45
   Ser Ser Ser Ser Ser Thr Arg Ala Ala Ser Thr Thr Ser Arg Val Ser
       50                  55                  60
   Pro Thr Thr Ser Arg Ser Ser Ser Ala Thr Pro Pro Pro Gly Ser Thr
   65                  70                  75                  80
   Thr Thr Arg Val Pro Pro Val Gly Ser Gly Thr Ala Thr Tyr Ser Gly
                   85                  90                  95
   Asn Pro Phe Val Gly Val Thr Pro Trp Ala Asn Ala Tyr Tyr Ala Ser
               100                 105                 110
   Glu Val Ser Ser Leu Ala Ile Pro Ser Leu Thr Gly Ala Met Ala Thr
   115 120 125
   Ala Ala Ala Ala Val Ala Lys Val Pro Ser Phe Met Trp Leu Asp Thr
   130 135 140
   Leu Asp Lys Thr Pro Leu Met Glu Gln Thr Leu Ala Asp Ile Arg Thr
   145 150 155 160
   Ala Asn Lys Asn Gly Gly Asn Tyr Ala Gly Gln Phe Val Val Tyr Asp
   165 170 175
   Leu Pro Asp Arg Asp Cys Ala Ala Ala Ala Ser Asn Gly Glu Tyr Ser
   180 185 190
   Ile Ala Asp Gly Gly Val Ala Lys Tyr Lys Asn Tyr Ile Asp Thr Ile
   195 200 205
   Arg Gln Ile Val Val Glu Tyr Ser Asp Ile Arg Thr Leu Leu Val Ile
   210 215 220
   Glu Pro Asp Ser Leu Ala Asn Leu Val Thr Asn Leu Gly Thr Pro Lys
   225 230 235 240
   Cys Ala Asn Ala Gln Ser Ala Tyr Leu Glu Cys Ile Asn Tyr Ala Val
   245 250 255
   Thr Gln Leu Asn Leu Pro Asn Val Ala Met Tyr Leu Asp Ala Gly His
   260 265 270
   Ala Gly Trp Leu Gly Trp Pro Ala Asn Gln Asp Pro Ala Ala Gln Leu
   275 280 285
   Phe Ala Asn Val Tyr Lys Asn Ala Ser Ser Pro Arg Ala Leu Arg Gly
   290 295 300
   Leu Ala Thr Asn Val Ala Asn Tyr Asn Gly Trp Asn Ile Thr Ser Pro
   305 310 315 320
   Pro Ser Tyr Thr Gln Gly Asn Ala Val Tyr Asn Glu Lys Leu Tyr Ile
   325 330 335
   His Ala Ile Gly Pro Leu Leu Ala Asn His Gly Trp Ser Asn Ala Phe
   340 345 350
   Phe Ile Thr Asp Gln Gly Arg Ser Gly Lys Gln Pro Thr Gly Gln Gln
   355 360 365
   Gln Trp Gly Asp Trp Cys Asn Val Ile Gly Thr Gly Phe Gly Ile Arg
       370                 375                 380
   Pro Ser Ala Asn Thr Gly Asp Ser Leu Leu Asp Ser Phe Val Trp Val
   385                 390                 395                 400
   Lys Pro Gly Gly Glu Cys Asp Gly Thr Ser Asp Thr Ser Ala Pro Arg
                   405                 410                 415
   Phe Asp Ser His Cys Ala Leu Pro Asp Ala Leu Gln Pro Ala Pro Glu
               420                 425                 430
   Ala Gly Thr Trp Phe Gln Ala Tyr Phe Glu Gln Leu Leu Thr Asn Ala
           435                 440                 445
   Asn Pro Ser Phe Leu
       450
<210> 27
   <211> 1353
   <212> DNA
   <213> Artificial Sequence
<220>
   <223> Clone 382A2
<400> 27
   gcccctcttg ttgaggagcg ccaggcttgc gctgcccaat gggcccagtg tggtggcttc 60
   agctggaatg gtgctacctg ctgccagtct ggtagctact gcagcaagat caacgactat 120
   tactctcagt gcattcctgg agaaggcccc gccacttcca agtcgagcac tcttcctgct 180
   tccaccacaa caactcagcc aacttccact tcgactgctg gaacctcttc cactaccaag 240
   cctcctccag ctggatcggg aaccgctacg tattcaggca acccttactc tggggtcaac 300
   ctttgggcca atagctatta ccgctctgaa gttaccaacc tcgctattcc taagttgagc 360
   ggagccatgg ccactgctgc agcaaaggtc gcagatgttc cctcttttca gtggatggat 420
   acttatgacc acatctcctt catggaggag tctttggccg acatccgcaa ggccaacaag 480
   gctggcggta actatgccgg acagtttgtg gtgtatgact tgccggatcg cgattgcgct 540
   gccgctgcct cgaatggcga atactctctt gacaaggatg gcaagaacaa atataaggcc 600
   tatatcgcca agattaaggg tattctccag gactattccg atacccggat cattctggtt 660
   attgagcctg actctcttgc caacatggtg accaacatga acgtcccaaa gtgtgccaat 720
   gctgcttcag cctacaagga gctcaccatt cacgccctca aggagctgaa ccttccaaat 780
   gtttccatgt atatcgacgc tggccatggt ggatggcttg gctggccggc aaaccttcct 840
   ccggccgctc agctatacgg tcagctctac aaggatgcag gcaagccgtc tcgccttcgc 900
   ggattggtca ccaatgtctc caactacaac gcctggaagc tatccagcaa gccagactac 960
   acggagagca accccaacta cgacgagcag aagtacatcc acgctctgtc tcctcttctt 1020
   gagcaggagg gctggcccgg tgccaagttc atcgtcgatc aaggtcgatc gggaaagcag 1080
   cctaccggac agaaggcttg gggagactgg tgcaatgctc ccggcaccgg atttggtctc 1140
   cgcccatccg caaacactgg ggacgccttg gtcgatgctt ttgtctgggt caagccaggc 1200
   ggcgagtctg acggcaccag cgacaccagt gcggctcgat acgactacca ctgtggtatt 1260
   gacggcgccg tcaagccggc gcctgaggct ggtacctggt tccaagccta ctttgagcag 1320
   cttctcaaga acgcaaaccc atcgttcctg taa 1353
<210> 28
   <211> 450
   <212> PRT
   <213> Artificial Sequence
<220>
   <223> Clone 382A2
<400> 28
   Ala Pro Leu Val Glu Glu Arg Gln Ala Cys Ala Ala Gln Trp Ala Gln
   1               5                   10                  15
   Cys Gly Gly Phe Ser Trp Asn Gly Ala Thr Cys Cys Gln Ser Gly Ser
               20                  25                  30
   Tyr Cys Ser Lys Ile Asn Asp Tyr Tyr Ser Gln Cys Ile Pro Gly Glu
           35                  40                  45
   Gly Pro Ala Thr Ser Lys Ser Ser Thr Leu Pro Ala Ser Thr Thr Thr
       50                  55                  60
   Thr Gln Pro Thr Ser Thr Ser Thr Ala Gly Thr Ser Ser Thr Thr Lys
   65                  70                  75                  80
   Pro Pro Pro Ala Gly Ser Gly Thr Ala Thr Tyr Ser Gly Asn Pro Tyr
                   85                  90                  95
   Ser Gly Val Asn Leu Trp Ala Asn Ser Tyr Tyr Arg Ser Glu Val Thr
               100                 105                 110
   Asn Leu Ala Ile Pro Lys Leu Ser Gly Ala Met Ala Thr Ala Ala Ala
           115                 120                 125
   Lys Val Ala Asp Val Pro Ser Phe Gln Trp Met Asp Thr Tyr Asp His
       130                 135                 140
   Ile Ser Phe Met Glu Glu Ser Leu Ala Asp Ile Arg Lys Ala Asn Lys
   145 150 155 160
   Ala Gly Gly Asn Tyr Ala Gly Gln Phe Val Val Tyr Asp Leu Pro Asp
   165 170 175
   Arg Asp Cys Ala Ala Ala Ala Ser Asn Gly Glu Tyr Ser Leu Asp Lys
   180 185 190
   Asp Gly Lys Asn Lys Tyr Lys Ala Tyr Ile Ala Lys Ile Lys Gly Ile
   195 200 205
   Leu Gln Asp Tyr Ser Asp Thr Arg Ile Ile Leu Val Ile Glu Pro Asp
   210 215 220
   Ser Leu Ala Asn Met Val Thr Asn Met Asn Val Pro Lys Cys Ala Asn
   225 230 235 240
   Ala Ala Ser Ala Tyr Lys Glu Leu Thr Ile His Ala Leu Lys Glu Leu
   245 250 255
   Asn Leu Pro Asn Val Ser Met Tyr Ile Asp Ala Gly His Gly Gly Trp
   260 265 270
   Leu Gly Trp Pro Ala Asn Leu Pro Pro Ala Ala Gln Leu Tyr Gly Gln
   275 280 285
   Leu Tyr Lys Asp Ala Gly Lys Pro Ser Arg Leu Arg Gly Leu Val Thr
   290 295 300
   Asn Val Ser Asn Tyr Asn Ala Trp Lys Leu Ser Ser Lys Pro Asp Tyr
   305 310 315 320
   Thr Glu Ser Asn Pro Asn Tyr Asp Glu Gln Lys Tyr Ile His Ala Leu
   325 330 335
   Ser Pro Leu Leu Glu Gln Glu Gly Trp Pro Gly Ala Lys Phe Ile Val
   340 345 350
   Asp Gln Gly Arg Ser Gly Lys Gln Pro Thr Gly Gln Lys Ala Trp Gly
   355 360 365
   Asp Trp Cys Asn Ala Pro Gly Thr Gly Phe Gly Leu Arg Pro Ser Ala
   370 375 380
   Asn Thr Gly Asp Ala Leu Val Asp Ala Phe Val Trp Val Lys Pro Gly
   385 390 395 400
   Gly Glu Ser Asp Gly Thr Ser Asp Thr Ser Ala Ala Arg Tyr Asp Tyr
   405 410 415
   His Cys Gly Ile Asp Gly Ala Val Lys Pro Ala Pro Glu Ala Gly Thr
   420 425 430
   Trp Phe Gln Ala Tyr Phe Glu Gln Leu Leu Lys Asn Ala Asn Pro Ser
   435 440 445
   Phe Leu
   450
<210> 29
   <211> 1323
   <212> DNA
   <213> Gibberella zeae
<400> 29
   gctcctgttg aagagcgtca gtcttgcagc aacggagtct ggtctcaatg tggtggtcag 60
   aactggtccg gtactccttg ctgcaccagt ggaaacaagt gtgtcaaggt caacgacttc 120
   tactcccagt gccagcctgg atctgcagac ccttctccca catccaccat tgtctccgcg 180
   acgaccacca aggctactac tactggtagt ggaggctctg tcacctcgcc tcctcctgtt 240
   gccaccaaca accctttttc tggggtcgat ctgtgggcca ataactatta ccgctctgaa 300
   gttagcactc tcgctattcc taagttgagc ggagccatgg ccactgctgc agcaaaggtc 360
   gcagatgttc cctcttttca gtggatggat acttatgacc acatctcctt catggaggag 420
   tctttggccg acatccgcaa ggccaacaag gctggcggta actatgccgg acagtttgtg 480
   gtgtatgact tgccggatcg cgattgcgct gccgctgcct cgaatggcga atactctctt 540
   gacaaggatg gcaagaacaa atataaggcc tatatcgcca agattaaggg tattctccag 600
   gactattccg atacccggat cattctggtt attgagcctg actctcttgc caacatggtg 660
   accaacatga acgtcccaaa gtgtgccaat gctgcttcag cctacaagga gctcaccatt 720
   cacgccctca aggagctgaa ccttccaaat gtttccatgt atatcgacgc tggccatggt 780
   ggatggcttg gctggccggc aaaccttcct ccggccgctc agctatacgg tcagctctac 840
   aaggatgcag gcaagccgtc tcgccttcgc ggattggtca ccaatgtctc caactacaac 900
   gcctggaagc tatccagcaa gccagactac acggagagca accccaacta cgacgagcag 960
   aagtacatcc acgctctgtc tcctcttctt gagcaggagg gctggcccgg tgccaagttc 1020
   atcgtcgatc aaggtcgatc gggaaagcag cctaccggac agaaggcttg gggagactgg 1080
   tgcaatgctc ccggcaccgg atttggtctc cgcccatccg caaacactgg ggacgccttg 1140
   gtcgatgctt ttgtctgggt caagccaggc ggcgagtctg acggcaccag cgacaccagt 1200
   gcggctcgat acgactacca ctgtggtatt gacggcgccg tcaagccggc gcctgaggct 1260
   ggtacctggt tccaagccta ctttgagcag cttctcaaga acgcaaaccc atcgttcctg 1320
   taa 1323
<210> 30
   <211> 440
   <212> PRT
   <213> Gibberella zeae
<400> 30
   Ala Pro Val Glu Glu Arg Gln Ser Cys Ser Asn Gly Val Trp Ser Gln
   1               5                   10                  15
   Cys Gly Gly Gln Asn Trp Ser Gly Thr Pro Cys Cys Thr Ser Gly Asn
               20                  25                  30
   Lys Cys Val Lys Val Asn Asp Phe Tyr Ser Gln Cys Gln Pro Gly Ser
           35                  40                  45
   Ala Asp Pro Ser Pro Thr Ser Thr Ile Val Ser Ala Thr Thr Thr Lys
       50                  55                  60
   Ala Thr Thr Thr Gly Ser Gly Gly Ser Val Thr Ser Pro Pro Pro Val
   65                  70                  75                  80
   Ala Thr Asn Asn Pro Phe Ser Gly Val Asp Leu Trp Ala Asn Asn Tyr
                   85                  90                  95
   Tyr Arg Ser Glu Val Ser Thr Leu Ala Ile Pro Lys Leu Ser Gly Ala
               100                 105                 110
   Met Ala Thr Ala Ala Ala Lys Val Ala Asp Val Pro Ser Phe Gln Trp
           115                 120                 125
   Met Asp Thr Tyr Asp His Ile Ser Phe Met Glu Glu Ser Leu Ala Asp
       130                 135                 140
   Ile Arg Lys Ala Asn Lys Ala Gly Gly Asn Tyr Ala Gly Gln Phe Val
   145                 150                 155                 160
   Val Tyr Asp Leu Pro Asp Arg Asp Cys Ala Ala Ala Ala Ser Asn Gly
                   165                 170                 175
   Glu Tyr Ser Leu Asp Lys Asp Gly Lys Asn Lys Tyr Lys Ala Tyr Ile
               180                 185                 190
   Ala Lys Ile Lys Gly Ile Leu Gln Asp Tyr Ser Asp Thr Arg Ile Ile
           195                 200                 205
   Leu Val Ile Glu Pro Asp Ser Leu Ala Asn Met Val Thr Asn Met Asn
       210                 215                 220
   Val Pro Lys Cys Ala Asn Ala Ala Ser Ala Tyr Lys Glu Leu Thr Ile
   225                 230                 235                 240
   His Ala Leu Lys Glu Leu Asn Leu Pro Asn Val Ser Met Tyr Ile Asp
                   245                 250                 255
   Ala Gly His Gly Gly Trp Leu Gly Trp Pro Ala Asn Leu Pro Pro Ala
               260                 265                 270
   Ala Gln Leu Tyr Gly Gln Leu Tyr Lys Asp Ala Gly Lys Pro Ser Arg
           275                 280                 285
   Leu Arg Gly Leu Val Thr Asn Val Ser Asn Tyr Asn Ala Trp Lys Leu
       290                 295                 300
   Ser Ser Lys Pro Asp Tyr Thr Glu Ser Asn Pro Asn Tyr Asp Glu Gln
   305                 310                 315                 320
   Lys Tyr Ile His Ala Leu Ser Pro Leu Leu Glu Gln Glu Gly Trp Pro
                   325                 330                 335
   Gly Ala Lys Phe Ile Val Asp Gln Gly Arg Ser Gly Lys Gln Pro Thr
               340                 345                 350
   Gly Gln Lys Ala Trp Gly Asp Trp Cys Asn Ala Pro Gly Thr Gly Phe
           355                 360                 365
   Gly Leu Arg Pro Ser Ala Asn Thr Gly Asp Ala Leu Val Asp Ala Phe
       370                 375                 380
   Val Trp Val Lys Pro Gly Gly Glu Ser Asp Gly Thr Ser Asp Thr Ser
   385                 390                 395                 400
   Ala Ala Arg Tyr Asp Tyr His Cys Gly Ile Asp Gly Ala Val Lys Pro
                   405                 410                 415
   Ala Pro Glu Ala Gly Thr Trp Phe Gln Ala Tyr Phe Glu Gln Leu Leu
               420                 425                 430
   Lys Asn Ala Asn Pro Ser Phe Leu
           435                 440
<210> 31
   <211> 1353
   <212> DNA
   <213> Nectria haematococca
<400> 31
   gcccctcttg ttgaggagcg ccaggcttgc gctgcccaat gggcccagtg tggtggcttc      60
   agctggaatg gtgctacctg ctgccagtct ggtagctact gcagcaagat caacgactat     120
   tactctcagt gcattcctgg agaaggcccc gccacttcca agtcgagcac tcttcctgct     180
   tccaccacaa caactcagcc aacttccact tcgactgctg gaacctcttc cactaccaag     240
   cctcctccag ctggatcggg aaccgctacg tattcaggca acccttactc tggggtcaac     300
   ctttgggcca atagctatta ccgctctgaa gttaccaacc tcgctattcc taagttgagc     360
   ggagccatgg ccactgctgc agcaaaggtc gcagatgttc cctcttatca gtggatggat     420
   tctttcgacc acatctccct catggaggac accttggtcg acatccgcaa ggccaacctg     480
   gctggcggta actatgccgg acagtttgtg gtgtatgact tgccggatcg cgattgcgct     540
   gccgccgcct cgaatggcga atactctctc gacaatgatg gcgccaacaa atataagaac     600
   tatatccaaa ccattaagaa gattatccag agctattccg atatccggat actcctggtt     660
   attgagcctg actctcttgc caacctggtg accaacatgg atgtcgccaa gtgtgccaag     720
   gctcatgatg cctacatcag cctgacgaac tacgccgtca cagaactgaa ccttccaaat     780
   gttgcgatgt atttggacgc tggccatgca ggatggcttg gctggccggc aaaccaaggc     840
   ccggccgcta aactatttgc aagcatctac aaggatgcag gcaagccggc cgctcttcgc     900
   ggattggcaa ccaatgtcgc caactacaac gcctggagcc tcagcagcgc tccaccttac     960
   acgcaaggcg cctctatcta cgacgagaag agcttcatcc acgctatggg acctcttctt    1020
   gagcagaatg gctggcctgg tgcccacttc atcactgatc aaggtcgatc gggaaagcag    1080
   cctaccggac agatccagtg gggagactgg tgcaattcca agggcaccgg atttggtatt    1140
   cgcccatccg caaacactgg ggactcgttg ctggatgctt ttgtctgggt caagccaggc    1200
   ggcgagtctg acggcaccag cgacaccagt gcgacccgat acgactacca ctgtggtgct    1260
   tctgccgcct tgcaaccggc gcctgaggct ggtacctggt tccaagccta ctttgagcag    1320
   cttctcacaa acgcaaaccc atcgttcctg taa                                1353
<210> 32
   <211> 450
   <212> PRT
   <213> Nectria haematococca
<400> 32
   Ala Pro Leu Val Glu Glu Arg Gln Ala Cys Ala Ala Gln Trp Ala Gln
   1               5                   10                  15
   Cys Gly Gly Phe Ser Trp Asn Gly Ala Thr Cys Cys Gln Ser Gly Ser
               20                  25                  30
   Tyr Cys Ser Lys Ile Asn Asp Tyr Tyr Ser Gln Cys Ile Pro Gly Glu
           35                  40                  45
   Gly Pro Ala Thr Ser Lys Ser Ser Thr Leu Pro Ala Ser Thr Thr Thr
       50                  55                  60
   Thr Gln Pro Thr Ser Thr Ser Thr Ala Gly Thr Ser Ser Thr Thr Lys
   65                  70                  75                  80
   Pro Pro Pro Ala Gly Ser Gly Thr Ala Thr Tyr Ser Gly Asn Pro Tyr
                   85                  90                  95
   Ser Gly Val Asn Leu Trp Ala Asn Ser Tyr Tyr Arg Ser Glu Val Thr
               100                 105                 110
   Asn Leu Ala Ile Pro Lys Leu Ser Gly Ala Met Ala Thr Ala Ala Ala
           115                 120                 125
   Lys Val Ala Asp Val Pro Ser Tyr Gln Trp Met Asp Ser Phe Asp His
       130                 135                 140
   Ile Ser Leu Met Glu Asp Thr Leu Val Asp Ile Arg Lys Ala Asn Leu
   145                 150                 155                 160
   Ala Gly Gly Asn Tyr Ala Gly Gln Phe Val Val Tyr Asp Leu Pro Asp
                   165                 170                 175
   Arg Asp Cys Ala Ala Ala Ala Ser Asn Gly Glu Tyr Ser Leu Asp Asn
               180                 185                 190
   Asp Gly Ala Asn Lys Tyr Lys Asn Tyr Ile Gln Thr Ile Lys Lys Ile
           195                 200                 205
   Ile Gln Ser Tyr Ser Asp Ile Arg Ile Leu Leu Val Ile Glu Pro Asp
       210                 215                 220
   Ser Leu Ala Asn Leu Val Thr Asn Met Asp Val Ala Lys Cys Ala Lys
   225                 230                 235                 240
   Ala His Asp Ala Tyr Ile Ser Leu Thr Asn Tyr Ala Val Thr Glu Leu
                   245                 250                 255
   Asn Leu Pro Asn Val Ala Met Tyr Leu Asp Ala Gly His Ala Gly Trp
               260                 265                 270
   Leu Gly Trp Pro Ala Asn Gln Gly Pro Ala Ala Lys Leu Phe Ala Ser
           275                 280                 285
   Ile Tyr Lys Asp Ala Gly Lys Pro Ala Ala Leu Arg Gly Leu Ala Thr
       290                 295                 300
   Asn Val Ala Asn Tyr Asn Ala Trp Ser Leu Ser Ser Ala Pro Pro Tyr
   305                 310                 315                 320
   Thr Gln Gly Ala Ser Ile Tyr Asp Glu Lys Ser Phe Ile His Ala Met
                   325                 330                 335
   Gly Pro Leu Leu Glu Gln Asn Gly Trp Pro Gly Ala His Phe Ile Thr
               340                 345                 350
   Asp Gln Gly Arg Ser Gly Lys Gln Pro Thr Gly Gln Ile Gln Trp Gly
           355                 360                 365
   Asp Trp Cys Asn Ser Lys Gly Thr Gly Phe Gly Ile Arg Pro Ser Ala
       370                 375                 380
   Asn Thr Gly Asp Ser Leu Leu Asp Ala Phe Val Trp Val Lys Pro Gly
   385                 390                 395                 400
   Gly Glu Ser Asp Gly Thr Ser Asp Thr Ser Ala Thr Arg Tyr Asp Tyr
                   405                 410                 415
   His Cys Gly Ala Ser Ala Ala Leu Gln Pro Ala Pro Glu Ala Gly Thr
               420                 425                 430
   Trp Phe Gln Ala Tyr Phe Glu Gln Leu Leu Thr Asn Ala Asn Pro Ser
           435                 440                 445
   Phe Leu
       450
<210> 33
   <211> 54
   <212> DNA
   <213> Artificial Sequence
<220>
   <223> peptide signal de CBH2
<400> 33
   atgattgtcg gcattctcac cacgctggct acgctggcca cactcgcagc tagt 54
<210> 34
   <211> 99
   <212> DNA
   <213> Saccharomyces cerevisiae
<400> 34
   atgaagcttt ccaccatcct tttcacagcc tgcgctaccc tggctgccgc cctcccttcc      60
   cccatcactc cttctgaggc cgcagttctg cagaagagg                              99
<210> 35
   <211> 33
   <212> PRT
   <213> Saccharomyces cerevisiae
<400> 35
   Met Lys Leu Ser Thr Ile Leu Phe Thr Ala Cys Ala Thr Leu Ala Ala
   1               5                   10                  15
   Ala Leu Pro Ser Pro Ile Thr Pro Ser Glu Ala Ala Val Leu Gln Lys
               20                  25                  30
   Arg